(19) Europäisches Patentamt
European Patent Office
Office européen des brevets

(11) Numéro de publication: **0 469 984 B1**

(12) **FASCICULE DE BREVET EUROPEEN**

(45) Date de publication du fascicule du brevet: **18.10.95**

(51) Int. Cl.$^6$: **C07D 209/42**, A61K 31/40, C07C 311/00

(21) Numéro de dépôt: **91402123.3**

(22) Date de dépôt: **30.07.91**

# (54) Dérivés de la N-sulfonyl indoline, leur préparation, les compositions pharmaceutiques en contenant.

(30) Priorité: **31.07.90 FR 9009778**

(43) Date de publication de la demande:
**05.02.92 Bulletin 92/06**

(45) Mention de la délivrance du brevet:
**18.10.95 Bulletin 95/42**

(84) Etats contractants désignés:
**AT BE CH DE DK ES FR GB GR IT LI LU NL SE**

(56) Documents cités:
**EP-A- 0 093 084**
**DE-A- 3 705 934**
**US-A- 3 838 167**

(73) Titulaire: **SANOFI**
**32-34, rue Marbeuf**
**F-75008 Paris (FR)**

(72) Inventeur: **Wagnon, Jean**
**90, rue des Galaxies,**
**Le Hameau de la Rauze**
**F-34000 Montpellier (FR)**
Inventeur: **de Cointet, Paul**
**6, rue Darquier**
**F-31000 Toulouse (FR)**
Inventeur: **Nisato, Dino**
**2, Rue de Terre Rouge**
**F-34680 Saint Georges d'Orques (FR)**
Inventeur: **Plouzane, Claude**
**5 Allée des Terres Rouges**
**F-34680 Saint Georges d'Orques (FR)**
Inventeur: **Serradeil-Legal, Claudine**
**Bâtiment B1,**
**Les Ormes**
**F-31320 Castanet-Tolosan (FR)**

(74) Mandataire: **Gillard, Marie-Louise et al**
**Cabinet Beau de Loménie**
**158, rue de l'Université**
**F-75340 Paris Cédex 07 (FR)**

**EP 0 469 984 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de la N-sulfonyl indoline, leur préparation et les compositions en contenant.

Le brevet US 3 838 167 décrit certains dérivés du N-sulfonyl indole répondant à la formule :

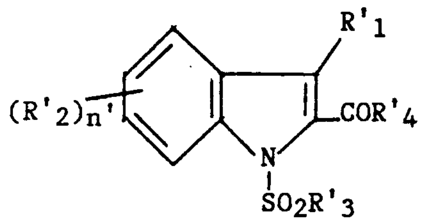

1

dans laquelle

- $R'_1$ représente l'hydrogène, un alkyle ou un phényle éventuellement substitué ;
- $R'_2$ représente un halogène, un alkyle, un alcoxy, un nitro ou trifluorométhyle ;
- $R'_3$ représente un alkyle, un phényle ou un alkylphényle ;
- $R'_4$ représente un alkyle, un phényle, éventuellement substitué, un alcoxy ou un phénoxy ;
- n′ = 0,1 ou 2.

Ces composés 1 sont des intermédiaires de synthèse pour la préparation de dérivés indoliques, actifs sur le système nerveux central, de formule :

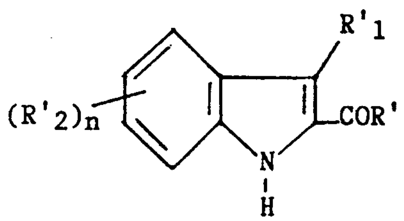

2

dans laquelle R′ représente un alkyle, un phényle éventuellement substitué ou un hydroxyle.

La demande de brevet EP-A-0 093 084 décrit des dérivés esters d'acide 1-carboxylalkanoylindoline 2-carboxylique de formule

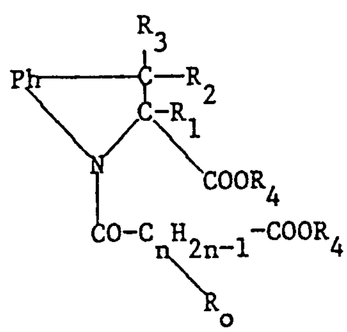

à activité hypotensive ou antihypertensive.

Des dérivés d'indoline de formule

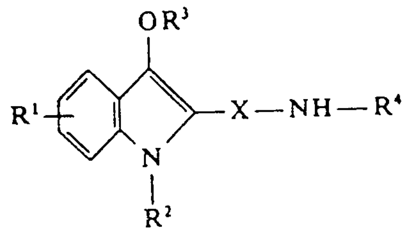

dans laquelle $R_2$ représente hydrogène, méthyl ou éthyl, sont décrits dans la demande de brevet DE-A-37 05 934. Ces dérivés ont une activité anxiolytique, sédative et anticonvulsive.

Les dérivés N-sulfonyl indoline selon la présente invention ont une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : $V_1$, $V_2$, $V_{1a}$, $V_{1b}$ et exerce ainsi des effets cardiovasculaires, centraux, hépatiques, antidiurétiques et agrégants. Les antagonistes des récepteurs de la vasopressine peuvent intervenir sur la régulation de la circulation centrale et périphérique, notamment les circulations coronaire, rénale et gastrique ; ainsi que le métabolisme de l'eau et la libération de l'hormone adrénocorticotrophique (ACTH). Les récepteurs de la vasopressine comme ceux de l'ocytocine se trouvent aussi sur la muscle lisse de l'utérus. L'ocytocine a une structure peptidique proche de celle de la vasopressine. Ses récepteurs se trouvent également sur les cellules myoépithéliales de la glande mammaire et dans le système nerveux central. (Presse Médicale, 1987, 16 (10), 481-485, J. Lab. Clin. Med., 1989, 114(6), 617-632 et Pharmacol. Rev., 1991, 43 (1), 73-108).

Ainsi les composés selon l'invention sont utiles notamment dans le traitement des affections du système nerveux central, du système cardiovasculaire et de la sphère gastrique chez l'homme et chez l'animal.

La présente invention a pour objet des composés de formule:

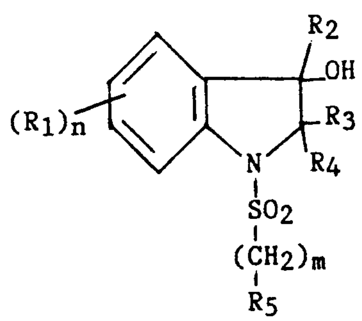

(I)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un alkyle en $C_1$-$C_4$ ; un naphtyl-1 ; un naphtyl-2 ; un phényle non substitué ou substitué par un ou plusieurs substituants choisis parmi un atome d'halogène, un alkyle en $C_1$-$C_4$, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy, un groupe cyano ;

- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6 ;

ainsi que leurs sels éventuels.

Les composés (I) présentent une isomérie cis-trans autour de la liaison 2,3 de l'indoline. Les différents isomères font partie intégrante de l'invention.

Par convention, on appelle isomère cis les composés (I) dans lesquels $R_2$ et $R_4$ sont du même coté du cycle.

Par convention, on appelle isomère trans les composés (I) dans lesquels $R_2$ et $R_4$ sont chacun d'un coté du cycle.

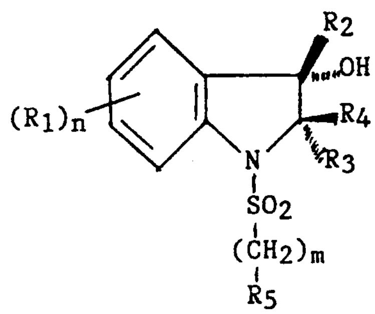

(I)

isomère cis

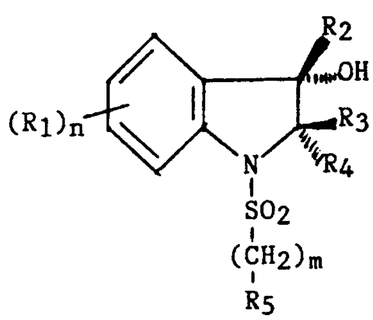

(I)

isomère trans

En outre les composés selon l'invention présentent 2 atomes de carbone asymétriques. Les isomères optiques des composés (I) font partie de l'invention.

Dans la présente description et dans les revendications qui vont suivre, on entend par halogène un atome de fluor, de chlore, de brome ou d'iode ; par groupe alkyle on entend des groupes hydrocarbonés linéaires ou ramifiés.

Des composés (I) préférés selon l'invention sont ceux dans lesquels au moins l'une des conditions suivantes est respectée :

- $R_1$ représente un atome de chlore, de brome ou un groupe méthoxy et n = 1 ;
- $R_2$ représente un chlorophényle, un méthoxyphényle ou un cyclohexyle ;
- $R_3$ est l'hydrogène ;
- $R_4$ représente un alcoxycarbonyle dans lequel le groupe alkyle est en $C_1$-$C_6$, ou bien $R_4$ représente un groupe carboxamide $NR_6R_7$ dans lequel $R_6$ et $R_7$ sont des alkyles en $C_1$-$C_6$ ;
- $R_5$ représente un phényle substitué en position 3 et 4, ou en position 2 et 4 par un groupe méthoxy, ou bien $R_5$ représente un phényle substitué en position 4 par un groupe méthyle ;
- m est 0.

Particulièrement préférés sont les composés (I) sous forme d'isomère cis

Dans la description et dans les exemples, les abréviations suivantes sont utilisées.

DCM : dichlorométhane
Ether iso : éther isopropylique
AcOEt : acétate d'éthyle
MeOH : méthanol
EtOH : éthanol
Ether : éther éthylique
DMF : diméthylformamide
THF : tétrahydrofuranne.
DMSO : diméthylsulfoxyde
DIPEA : diisopropyléthylamine
DBU : 1-8 diazabicyclo [5.4.0] undec-7-ene
TBD : triaza-1,5,7 bicyclo [4.4.0] dec-5-ene
DBN : diaza-1,5 bicyclo [4.3.0] non-5-ene
DMAP : diméthylamino-4 pyridine
DMPU : diméthyl-1,3 oxo-2 hexahydropyrimidinone
TMEDA : tétraméthyléthylènediamine
LDA : lithium diisopropylamide
HMPA : hexaméthylphosphoramide
HMDS : hexaméthyl 1,1,1,3,3,3,-disilazane.
BOP : hexafluorophosphate de benzotriazolyloxy trisdiméthylamino phosphonium.
Fc : Point de fusion
Solution saline : eau saturée en chlorure de sodium
Carboglace : dioxyde de carbone solide
CCM : chromatographie en couche mince
HPLC : chromatographie liquide à haute pression
RMN : résonnance magnétique nucléaire
s : singulet
m : multiplet
s.e. : singulet élargi
d : doublet
Eau chlorhydrique : acide chlorhydrique dilué, environ 1N
NaH à 80 % : dispersion d'hydrure de sodium dans l'huile minérale (Janssen Chemica)
Me = Méthyl
Et : Ethyl
Ph : Phényl
TA : température ambiante.

La présente invention a également pour objet le procédé de préparation des composés (I).

Ce procédé est caractérisé en ce que

a) on fait réagir sur un dérivé d'amino-2 phénone de formule:

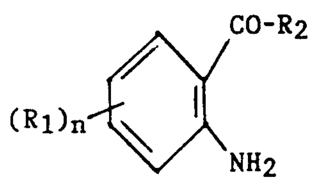

(II)

dans laquelle $R_1$, $R_2$ et n ont les significations indiquées ci-dessus pour I, un dérivé sulfonyle de formule :

$Hal - SO_2 - (CH_2)_m - R_5$ (III)

dans laquelle

- Hal représente un halogène, de préférence le chlore ou le brome,
- et $R_5$ a la signification indiquée ci-dessus pour (I) ;

b) le composé ainsi obtenu de formule

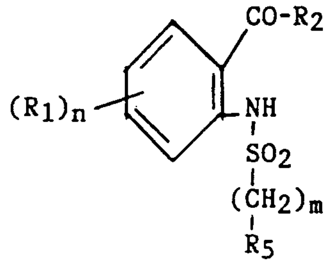

(IV)

est traité par un dérivé halogéné de formule :

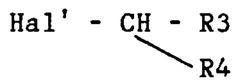

(V)

dans laquelle

Hal' représente un halogène préférentiellement le brome et $R_3$ et $R_4$ ont les significations indiquées ci-dessus pour I ;

c) le composé ainsi obtenu de formule:

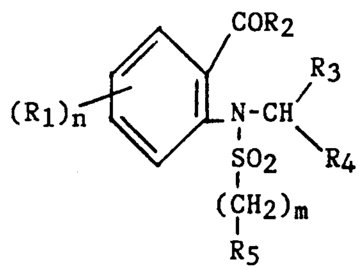

(VI)

est cyclisé en milieu basique pour préparer le composé (I) selon l'invention ;

d) on sépare éventuellement les isomères cis et trans du composé (I).

Les dérivés d'amino-2 phénone (II) sont connus ou préparés par des méthodes connues, telles que celles décrites par A.K. Singh et al., Synth Commun. 1986, 16 (4), 485 et G.N. Walker J. Org. Chem., 1962, 27, 1929.

Les dérivés halogénosulfonyle (III) sont connus ou préparés par des méthodes connues. Ainsi par exemple, le chlorure de diméthylamino-4 phénylsulfonyle est préparé selon C.N. SUKENIK et al., J. Amer. Chem. Soc., 1977, 99, 851-858 ; le chlorure de p-benzyloxysulfonyle est préparé selon la demande de brevet européen EP 229 566.

Le chlorure d'alkyloxy sulfonyle est préparé à partir de l'alkyloxy sulfonate de sodium, lui-même préparé par action d'un halogénure d'alkyle sur l'hydroxyphénylsulfonate de sodium. Les amino-2 trifluorométhyl-2 benzophénones et les autres dérivés trifluorométhylés sont préparés selon le brevet US 3 341 592.

Le chlorure de diméthoxy-2,4 benzylsulfonyle est préparé selon J. Am Chem. Soc., 1952, 74, 2008.

Les dérivés halogénés (V) sont connus ou préparés par des méthodes connues, telles que celles décrites par A.I. Vogel : a Text Book of Practical Organic Chemistry : Longman, 3rd ed. 1956, p. 383, ou G. Kirchner et al., J. Am. Chem. Soc., 1985, 107, 24, 7072.

L'étape a) du procédé est réalisée dans la pyridine par chauffage à une température comprise entre la température ambiante et le point d'ébullition du solvant pendant une période de temps comprise entre quelques heures et quelques jours. On peut éventuellement opérer en présence de diméthylaminopyridine que l'on utilise en quantité catalytique ou stoechiométrique.

L'étape b) du procédé est réalisée entre le sulfonamide (IV) et le dérivé halogéné en excès dans un solvant tel que le diméthylformamide ou le diméthylsulfoxyde, sous une atmosphère inerte, à une

température comprise entre 0 °C et la température ambiante, pendant un temps compris entre quelques heures et 24 heures, en présence d'hydrure de sodium.

L'étape c) du procédé s'apparente à une réaction d'aldolisation : le groupement $CH-R_3$ en $\alpha$ de l'ester ou de l'amide est déprotoné, puis la fonction carbonyle de la phénone agit comme un électrophile interne, ce qui conduit à la cyclisation avec apparition de deux carbones asymétriques (C*) .

On peut schématiser la réaction comme suit:

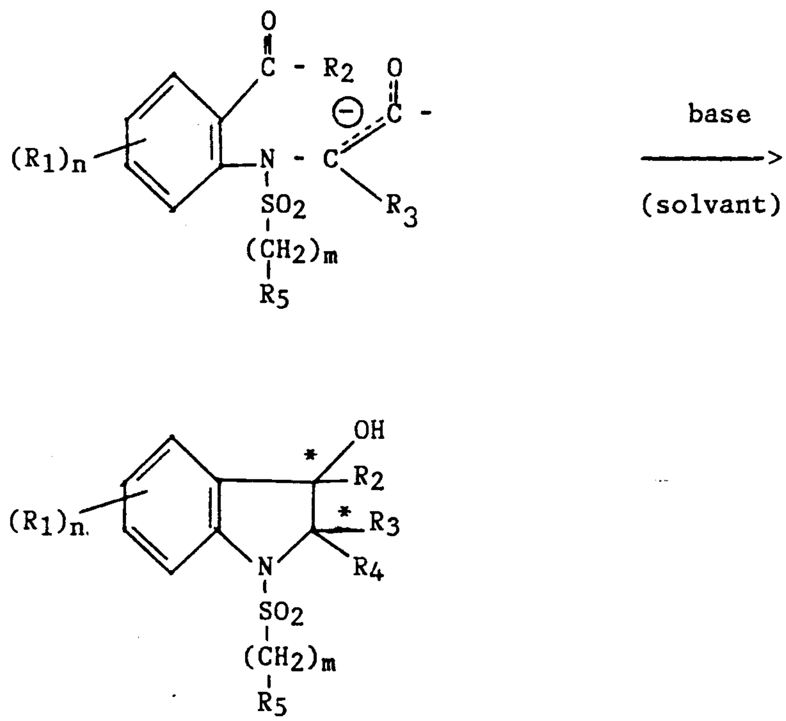


Les principes de la réaction d'addition aldolique ont été revus par C.H. Heathcock dans Asymmetric Synthesis,vol. 3 : Stereodifferentiating additions reactions, part B, 111-212 ; Academic Press, 1984, J. D. Morrison ed.

Il est connu que l'addition aldolique d'anions d'esters (ou d'anions d'amides) achiraux avec des bases achirales conduit à la formation de 2 diastéréoisomères racémiques de $\beta$-hydroxy esters (ou de $\beta$-hydroxy amides) dans un rapport qui dépend largement des conditions expérimentales utilisés. Parmi ces conditions, on peut citer : la nature de la base minérale ou organique utilisée, la nature des cations ou contre-ions, la présence éventuelle d'additifs dans le milieu réactionnel, le solvant, la température de la réaction, ainsi que la structure du composé qui subit cette réaction.

Lorsque $R_4$ représente un groupe carboxamide $CONR_6R_7$ dans lequel $R_6$ et $R_7$ ont les définitions données ci-dessus pour (I), on peut utiliser la soude dans l'eau, en présence d'un co-solvant, avec ou sans l'ajout d'un catalyseur de transfert de phase.

Pour effectuer cette réaction, on peut utiliser des bases organiques, par exemple:

- des bases organiques tertiaires comme la triéthylamine,
- des guanidines comme le triaza-1,5,7 bicyclo [4.4.0] dec-5-ène,
- des amidines comme diaza-1,8 bicyclo [5.4.0] undec-5-ène ou le diaza-1,5 bicyclo [4.3.0] non-5-ène,

dans un solvant ou un mélange de solvants choisis parmi , par exemple, le benzène, le THF, le dichlorométhane, le méthanol, le diméthylformamide ; la réaction est effectuée sous atmosphère inerte entre 25 et 110 °C ; la quantité de base utilisée est au moins stoechiométrique ; on peut également effectuer la réaction sans solvant, à la température du bain.

On peut également utiliser un alcoolate d'alcool primaire, secondaire ou tertiaire avec le lithium, le sodium, le potassium, le calcium ou le magnésium.

L'alcoolate est utilisé en quantité catalytique ou stoechiométrique dans un solvant anhydre, par exemple un alcool (éventuellement en présence d'un cosolvant tel que le THF), ou bien, en quantité stoechiométrique, dans le THF,le DMF ou le DMSO, éventuellement en présence d'éthers-couronne, par exemple le dicyclohexyl-18 crown-6 ; la réaction est effectuée entre 0° et 80 °C. On peut aussi utiliser une base

comme l'hydrure de sodium, l'hydrure de lithium ou l'hydrure de potassium, dans un solvant tel que par exemple l'éther éthylique, le THF, le benzène, le DMF, ou bien des amidures alcalins utilisés dans un solvant tel que l'ammoniaque, l'éther ou le toluène, à une température comprise entre -30 °C et 110 °C.

L'emploi d'amidure de type RR′NLi ou RR′NMgBr, dans lesquels R et R′ sont des radicaux monovalents, comme agent de déprotonation est une méthode de formation d'énolates d'esters ou d'amides, intermédiaires de la réaction d'aldolisation ; cette méthode a été revue récemment par R.E. Ireland et al., J. Org. Chem., 1991, 56, 650. Le solvant de la réaction peut être le benzène, l'hexane ou le THF, utilisé anhydre, sous atmosphère inerte. On peut ajouter des adjuvants comme LiF, LiCl, LiBr, LiI, LiBu, TMEDA, DMPU, HMPA ou éther couronne. (M. Murakate et al., J. Chem. Soc. Chem. Commun., 1990, 1657). On a étudié l'influence des conditions réactionnelles sur la proportion de chacun des isomères formés. A titre d'exemple, on peut citer l'utilisation du diisopropylamidure de lithium à -78 °C dans le THF anhydre, sous atmosphère inerte ou dans le THF en présence d'additifs tels que la tétraméthylènediamine, DMPU ou HMPA par exemple. D'autres amidures connus et utilisables sont, par exemple, le cyclohexylamidure de lithium, le tétraméthyl-2,2,6,6 cyclohexyl amidure de lithium.On peut également préparer d'autres amidures, par action de la quantité nécessaire de butyllithium dans l'hexane, sur des amines linéaires ou cycliques la réaction se faisant dans l'un des solvants cités ci-dessus. Enfin diverses publications décrivent des amidures d'amines secondaires optiquement actives : L. Duhamel et al., Bull. Soc. Chim., France, 1984, II, 421 ; J.K. Whitesell et al., J. Org. Chem., 1980, 45, 755 ; M. Murakata et al., J. Chem. Soc. Chem. Comm., 1990, 1657 ; M. Yamaguchi, Tetrahedron Lett., 1986, 27 (8), 959 ; P.J. Cox et N.S. Simpkins, Tetrahedron : Asymmetry, 1991, 2 (1), 1.

Les silylamidures de lithium, de sodium ou de potassium constituent un autre groupe de bases utilisables parmi lesquelles on peut citer : $(Me_3Si)_2NLi$, $(Me_2PhSi)_2NLi$, $(Et_3Li)_2NLi$, $(Me_3Si)_2NK$, $(Me_3Si)_2NNa$.

On peut également utiliser des amidures mixtes tels que décrits par Y. Yamamoto, Tetrahedron, 1990, 46, 4563, par exemple, le sel de lithium de N-(triméthylsilyl) benzylamine, ou un analogue dans lequel la benzylamine est remplacée par une amine primaire chirale comme la (R) ou la (S) $\alpha$-méthylbenzylamine.

Quand des amidures chiraux sont utilisés, les 2 diastéréoisomères cis et trans peuvent présenter ensemble ou indépendamment l'un et l'autre une activité optique créée par induction asymétrique et enrichissement énantiomérique. On détermine alors la proportion de chacun des énantiomères par dosage sur une colonne HPLC chirale.

A l'étape d) les 2 isomères du composé (I) formés sont extraits par les méthodes classiques et séparés par chromatographie ou cristallisation fractionnée.

Eventuellement, on sépare les isomères optiques de chacun des isomères cis et trans, par exemple par chromatographie préparative sur une colonne chirale.

Lorsque les 2 isomères des composés (I) sont difficilement séparables par les méthodes habituelles, on peut également préparer un composé de formule:

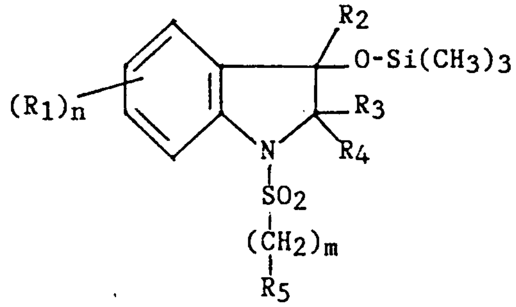

(VII)

dans laquelle $R_1$, $R_2$, $R_3$, $R_4$, $R_5$, m et n ont les significations indiquées ci-dessus pour I, par action de l'hexaméthyldisilazane sur le composé (I) correspondant. La réaction est effectuée en présence d'une quantité catalytique d'imidazole par chauffage à 60-120 °C sous atmosphère inerte. L'ester silylé est obtenu par cristallisation dans le milieu ou après chromatographie. Les 2 isomères de (VII) sont séparés par chromatographie sur silice, puis on hydrolyse chaque isomère de (VII) en milieu alcalin pour obtenir chaque isomère de (I).

Pour différencier et caractériser l'isomère cis et l'isomère trans d'un composé (I), plusieurs méthodes sont possibles. Lorsque $R_3$ est l'hydrogène, on effectue une analyse comparative par RMN à haut champ (250 MHz), assortie par exemple de l'étude de l'effet Overhauser (N.O.E.) entre, par exemple, le proton de

l'indoline ($R_3$ = H) et le proton de l'hydroxyle.

Lorsque $R_4$ représente un groupe carboxamide, les spectres IR de l'isomère cis et de l'isomère trans en solution dans le DCM sont différents. L'isomère cis présente le plus souvent une forte bande d'absorption fine et symétrique vers 3550-3520 $cm^{-1}$, due à la vibration de l'hydroxyle, alors que l'isomère trans ne présente aucune bande de vibration résolue dans cette région.

Grâce aux données recueillies, on a constaté que l'isomère cis est en général le plus mobile en CCM sur une plaque d'oxyde d'alumine (60F254 neutral, Type E, Merck), en éluant par du DCM contenant des proportions variables d'AcOEt. De même, par chromatographie sur colonne d'alumine (oxyde d'alumine 90, granulométrie 0,063-0,200 mm), l'isomère cis est élué le plus souvent le premier, l'éluant étant du DCM contenant des proportions variables d'AcOEt ou de MeOH).

Lorsque $R_4$ est un groupement ester, on peut effectuer la CCM sur une plaque de silice (Kieselgel 60F250, Merck) en éluant par le DCM, généralement l'isomère cis est le plus mobile, lorsque la CCM est réalisée sur le mélange des isomères.

Ainsi l'isomérie cis ou trans d'un composé (I) selon l'invention peut le plus souvent être déterminée par une méthode analytique. On procède également par analogie entre des composés voisins ou entre des composés préparés l'un à partir de l'autre.

Les composés (I) dans lesquels $R_4$ représente un groupe carboxy sont préparés par débenzylation des composés (I) dans lesquels $R_4$ représente un groupe benzyloxycarbonyle en effectuant une hydrogénation catalytique, par exemple en présence de palladium sur charbon.

Les composés (I) dans lesquels $R_4$ représente un groupe carboxamide $CONH_2$ peuvent être préparés à partir des composés (I) correspondants dans lesquels $R_4$ représente un groupe carboxy par exemple, par une méthode de couplage classique de la synthèse peptidique, par exemple en présence de BOP et de DIPEA.

On peut préparer un composé (I) dans lequel $R_1$ est un groupe amino et/ou un composé dans lequel $R_5$ représente un groupe phényle substitué par un amino, par transformation d'un composé (VI) obtenu à l'étape b) dans lequel $R_1$ est un groupe nitro et/ou $R_5$ est un groupe phényle substitué par un nitro, les autres substituants ayant les valeurs souhaitées pour (I), par hydrogénation catalytique, par exemple en présence de palladium sur charbon ou de rhodium sur alumine.

Les composés de formule (VI) obtenus à l'issue de l'étape b), dans lesquels $R_5$ représente un naphtyl-1; un naphtyl-2 ; un phényle substitué par un ou plusieurs substituants choisis parmi un atome d'halogène, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy ou un groupe cyano, sont nouveaux et font partie de l'invention .

L'affinité des composés selon l'invention pour les récepteurs de la vasopressine a été déterminée in vitro en utilisant la méthode décrite dans J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à nanomolaires.

De même, l'affinité des composés (I) selon l'invention pour les récepteurs de l'ocytocine a été déterminée in vitro par déplacement de l'ocytocine tritiée fixée aux récepteurs d'une préparation membranaire de glandes de rates gestantes. Les $CI_{50}$ des composés selon l'invention sont faibles, allant jusqu'à $10^{-7}$ M.

Par ailleurs, on a mesuré l'inhibition de l'agrégation plaquettaire induite par la vasopressine sur un plasma humain riche en plaquettes (PRP humain) en utilisant la méthode décrite dans Thrombosis Res., 1987, 45, 7-16. Les composés selon l'invention inhibent l'agrégation induite par la vasopressine 50 à 100 nM avec des $DI_{50}$ (doses inhibitrices) faibles, allant jusqu'à $10^{-8}$ M. Ces résultats montrent l'activité antagoniste des récepteurs $V_1$ des composés selon l'invention.

L'affinité des composés (I) selon l'invention pour les récepteurs $V_2$ a été mesurée selon une méthode adaptée de P. Crause et al., Molecular and Cellular Endocrinology, 1982, 28, 529-541.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes, notamment les affections cardiovasculaires, comme l'hypertension, l'insuffisance cardiaque, ou le vasospasme coronaire, en particulier chez le fumeur ; les affections du système nerveux central, les oedèmes cérébraux, les états psychotiques, les troubles de la mémoire par exemple ; les affections du système rénal comme le vasospasme rénal, la nécrose du cortex rénal ; les affections du système gastrique, par exemple les ulcères ou encore le syndrome de la sécrétion

inappropriée de l'hormone antidiurétique (SIADH). Chez la femme, les composés selon l'invention peuvent également être utilisés pour traiter la dysménorrhée ou le travail prématuré.

La présente invention a également pour objet des compositions pharmaceutiques contenant une dose efficace d'un composé selon l'invention ou d'un sel pharmaceutiquement acceptable et des excipients convenables. Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un des sels pharmaceutiquement acceptables, d'autres principes actifs qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention.

EXEMPLES 1 ET 2

Chloro-5 cyclohexyl-3 hydroxy-3 (naphtyl-1 sulfonyl)-1 indoline-2 carboxylate de méthyle, isomère cis, isomère trans.

A) chloro-5 [(naphtyl-1 sulfonyl) amino]-2 cyclohexylphénone.

On chauffe dans la pyridine à 100 ° C pendant 8 heures un mélange contenant 3 g d'amino-2 chloro-5 cyclohexylphénone et 3,2 g de chlorure de naphtyl-1 sulfonyle. On évapore la pyridine, ajoute de l'eau, extrait par de l'acétate d'éthyle puis filtre sur silice en éluant par du dichlorométhane. On obtient 4,27 g du produit attendu.

Après recristallisation dans un mélange DCM-éther iso,

Fc = 140-142 ° C.

B) chloro-5 [(N-méthoxycarbonylméthyl) (N-naphtyl-1 sulfonyl) amino]-2 cyclohexylphénone.

On dissout 4,27 g du produit obtenu à l'étape précédente dans 20 ml de DMF anhydre sous argon. On ajoute à 0 ° C, 320 mg d'hydrure de sodium à 80 % puis après 20 minutes, on ajoute en 30 minutes, 6,1 g de bromacétate d'éthyle, et on laisse 3 heures sous agitation à température ambiante. Après extraction, le produit brut obtenu est recristallisé dans un mélange DCM-éther iso, pour donner 2,45 g du composé attendu.

Fc = 130-132 ° C.

C) chloro-5 cyclohexyl-3 hydroxy-3 (naphtyl-1 sulfonyl)-1 indoline-2 carboxylate de méthyle.

On place 2,4 g du composé obtenu à l'étape précédente en suspension dans 30 ml de méthanol sous atmosphère d'azote et l'on ajoute à 0 ° C, 26 mg de méthylate de sodium et après 10 minutes à température ambiante, à nouveau 26 mg de méthylate de sodium, enfin après 45 mn, on ajoute 1 ml de THF pour achever la dissolution. Puis, après 1 heure, on forme un précipité par addition de neige carbonique et d'eau. Le précipité est filtré, repris par de l'acétate d'éthyle, lavé par de l'eau, de l'eau saline et séché. L'huile obtenue est chromatographiée sur silice, en éluant par DCM puis DCM contenant jusqu'à 10 % d'AcOEt, on sépare ainsi les 2 isomères :

Les composés contenus dans chacunes des fractions sont ensuite recristallisés dans un mélange DCM-éther iso

Fc = 155-157 ° C : isomère cis

Fc = 141-142 ° C : isomère trans

EXEMPLES 3 ET 4

Chloro-5 (fluoro-2 phényl)-3 hydroxy-3 (nitro-4 phénylsulfonyl)-1 indoline-2 carboxylate de méthyle, isomère cis, isomère trans.

A) chloro-5 fluoro-2′ [(nitro-4 phénylsulfonyl) amino]-2 benzophénone.

On chauffe à reflux pendant 10 heures dans la pyridine un mélange contenant 24,9 g d'amino-2 chloro-5 fluoro-2′ benzophénone et 22,1 g de chlorure de nitro-4 phénylsulfonyle. On évapore à siccité puis on ajoute de l'eau et de l'acétate d'éthyle, lave par de l'eau, de l'eau saline, sèche sur sulfate de magnésium, et évapore sous vide. Le produit attendu précipite à l'évaporation, on le filtre et le recristallise dans un mélange DCM-éther iso. On obtient 20 g.

Fc = 155 ° C.

B) chloro-5 fluoro-2′ [N-(méthoxycarbonylméthyl) (N-nitro-4 phénylsulfonyl) amino]-2 benzophénone.

On dissout 5 g du composé obtenu à l'étape précédente dans 20 ml de DMF à 0 ° C sous argon, et on ajoute 367 mg d'hydrure de sodium à 80 % et après 5 minutes, 3,5 g de bromacétate de méthyle, et à nouveau 3,5 g de bromacétate de méthyle après 1 heure. Après 5 heures à température ambiante, on verse le mélange réactionnel sur de l'eau glacée puis on extrait 3 fois par de l'acétate d'éthyle, lave 3 fois par de l'eau, de l'eau saline puis on sèche sur sulfate de magnésium. On chromatographie sur gel de silice en éluant par du DCM pour obtenir 6,1 g du composé attendu qui concrétise dans le méthanol.

C) 3 g du composé obtenu à l'étape précédente sont mis en suspension dans 50 ml de méthanol et refroidis dans un bain de glace. On ajoute 330 mg (0,1 équivalent) de méthylate de sodium et on laisse sous agitation pendant 60 minutes en laissant remonter la température à 10 ° C. On ajoute de la neige carbonique puis de l'eau et extrait 3 fois par de l'acétate d'éthyle, puis on lave à l'eau, à l'eau saline, sèche et évapore sous vide. Le brut de réaction (6,1 g) est chromatographié sur une colonne de silice préparée dans le DCM.

Par élution avec le DCM, on élue successivement les 2 isomères.

Isomère le moins polaire : isomère cis.

Après recristallisation dans un mélange DCM-éther iso,

Fc = 219-220 ° C

Isomère le plus polaire : isomère trans.

Après recristallisation dans un mélange DCM-méthanol,

Fc = 203-204 ° C.

EXEMPLES 5 ET 6

(Amino-4 phénylsulfonyl)-1 chloro-5 (fluoro-2 phényl)-3 hydroxy-3 indoline-2 carboxylate de méthyle, isomère trans, isomère cis.

A) Chloro-5 fluoro-2′ [N-méthoxycarbonylméthyl N-(amino-4) phénylsulfonyl] amino-2 benzophénone.

Le chloro-5 fluoro-2′ [N-méthoxycarbonylméthyl N-(nitro-4) phénylsulfonyl] amino-2 benzophénone préparé à l'exemple 2, étape b, est dissous dans 100 ml d'acétate d'éthyle et 5 ml de méthanol et on hydrogène à pression ordinaire, pendant 2 heures, en présence de 620 mg de palladium sur charbon à 10%, on observe en CCM l'existence de 3 composés (produit de départ, produit intermédiaire et produit attendu). On filtre le catalyseur, evapore le solvant et chromatographie sur gel de silice. Le composé attendu est élué par DCM contenant 1% de méthanol. Après recristallisation dans DCM-éther iso.

Fc = 168-170 °C.

B) isomère trans

On dissout 3,4 g du composé obtenu à l'étape précédente dans 20 ml de méthanol et 20 ml de THF à 0 °C, sous azote et on ajoute 190 mg de méthylate de sodium. Après 60 minutes sous agitation à 5 °C, on verse le milieu réactionnel dans l'eau et extrait par l'acétate d'éthyle. Un composé cristallise, il est recristallisé dans le DCM contenant un peu de méthanol.

Fc = 215-216 °C.

Il s'agit du composé trans, d'après l'étude du spectre RMN, effet NOE.

C) isomère cis

200 mg du composé 2 préparé à l'exemple 3 sont dissous dans 10 ml d'acétate d'éthyle et 2 ml de méthanol et on hydrogène à pression ordinaire en présence de 50 mg de Palladium sur charbon à 10 % pendant 3 heures. On filtre le catalyseur, évapore à siccité, chromatographie sur gel de silice en éluant par DCM contenant 1 % de méthanol. Le produit obtenu est recristallisé dans MeOH-éther iso. On obtient 105 mg.

Fc = 186-190 °C.

EXEMPLES 7 ET 8

(Fluoro-2 phényl)-3 hydroxy-3 nitro-5 tosyl-1 indoline-2 carboxylate de méthyle, isomère cis, isomère trans.

A) Fluoro-2′ nitro-5 tosylamino-2 benzophénone.

On porte au reflux dans 50 ml de pyridine pendant 24 heures un mélange contenant 10 g d'amino-2 nitro-5 fluoro-2′ benzophénone et 7,5 g de chlorure de tosyle. On évapore le solvant à siccité, ajoute de l'eau et de l'acétate d'éthyle, filtre un insoluble, lave la phase organique par une solution d'acide chlorhydrique diluée, de l'eau, de l'eau saline, puis sèche sur sulfate de magnésium et évapore. Le résidu est chromatographié sur silice et l'on élue le produit attendu par du DCM.

B) Fluoro-2′ [(N-méthoxycarbonylméthyl N-tosyl) amino]-2 nitro-5 benzophénone.

4 g du composé obtenu à l'étape précédente sont placés dans 40 ml de DMF anhydre et traités à 0 °C par 320 mg d'hydrure de sodium à 80 % et, après 10 minutes, par 6 g de bromacétate de méthyle. On laisse revenir à température ambiante, ajoute de l'eau et extrait par de l'acétate d'éthyle. Le résidu obtenu après évaporation du solvant est purifié par chromatographie sur gel de silice. L'huile obtenue par élution avec DCM puis DCM contenant jusqu'à 2 % d'AcOEt se solidifie entierement.

Analyse élémentaire : $C_{23}H_{19}FN_2O_7S$

| | | | |
|---|---|---|---|
| calculé % | C : 56,79 | H : 3,94 | N : 5,76 |
| trouvé % | C : 56,54 | H: 3,88 | N : 5,54 |

C) Une suspension contenant 1,70 g du produit obtenu à l'étape précédente dans 30 ml de méthanol est refroidie à 10 °C sous argon puis traitée par 100 mg de méthylate de sodium pendant 45 minutes. On ajoute un grand volume d'eau, extrait par de l'acétate d'éthyle, lave la phase organique par de l'eau jusqu'à neutralité, lave par une solution saline, sèche sur sulfate de magnésium et évapore sous vide. Le résidu est chromatographié sur gel de silice. Le composé le moins polaire est élué par DCM pur ; on obtient 700 mg : isomère cis.

Fc = 191-192 ° C. Après recristallisation (DCM-éther iso)

Le composé le plus polaire est élué par un mélange DCM-acétate d'éthyle (95/5, v/v). Après recristallisation par DCM-éther iso, on obtient 650 mg.

Fc = 206-207 ° C : isomère trans.

EXEMPLE 9

Amino-5 (fluoro-2 phényl)-3 hydroxy-3 tosyl-1 indoline-2 carboxylate de méthyle : isomère cis.

450 mg du composé cis, obtenu à l'exemple 4, sont solubilisés dans 13 ml d'un mélange acétate d'éthyle-méthanol (10/3, v/v) et hydrogénés à pression et température ordinaires en présence de 100 mg de Palladium sur charbon à 10 % pendant 2 heures. On filtre le catalyseur, évapore le solvant et chromatographie le résidu sur gel de silice en éluant par un mélange DCM/acétate d'éthyle (1/1, v/v).

Après trituration dans un mélange DCM-éther iso, on obtient une poudre blanche.

Fc = 203 ° C (isomère cis).

EXEMPLES 10 ET 11

Chloro-5 cyclohexyl-3 hydroxy-3 (méthoxy-4 phénylsulfonyl)-1 indoline-2 carboxylate de méthyle, isomère cis, isomère trans.

A) chloro-5 [(méthoxy-4 phénylsulfonyl) amino]-1 cyclohexylphénone.

On chauffe à 100 ° C pendant une nuit dans la pyridine un mélange contenant 20 g d'(amino-2 chloro-5) phényl cyclohexyl cétone et 18 g de chlorure de méthoxy-4 phénylsulfonyle, on concentre le solvant, reprend par de l'eau chlorhydrique, extrait au DCM, sèche et concentre. Le résidu est recristallisé dans un mélange éther iso/cyclohexane, on recueille 27 g du composé attendu qui cristallise.

Fc = 78-80 ° C.

B) chloro-5 [(N-méthoxycarbonylméthyl N-méthoxy-4 phénylsulfonyl) amino]-1 phénylcyclohexylcétone.

Le composé obtenu à l'étape précédente (27 g) est traité par 2,2 g d'hydrure de sodium dans 150 ml de DMF, à température ambiante, sous argon, pendant 30 minutes. On ajoute 50 g de bromure d'acétate de méthyle et laisse une nuit sous agitation. On évapore le DMF, reprend par de l'eau, extrait au DCM, sèche et concentre. Le résidu est chromatographié sur silice, en éluant par DCM, on receuille 19,5 g du composé attendu qui cristallise dans le méthanol.

Fc = 115-116 ° C.

C) Le produit obtenu à l'étape précédente (10 g) est solubilisé dans 350 ml de méthanol et on ajoute 0,6 g de méthylate de sodium dans 50 ml de méthanol. Après 5 minutes de contact, on voit par CCM que la réaction est terminée. On évapore le méthanol après avoir ajouté au milieu de la carboglace. On extrait par de l'eau, extrait au chlorure de méthylène et on effectue une chromatographie sur silice en éluant au chlorure de méthylène.

Les premières fractions contiennent l'isomère le moins polaire qui est recristallisé dans l'éther isopropylique.

Fc = 100 ° C (isomère cis).

On recueille ensuite l'isomère le plus polaire.

Fc = 145 ° C (isomère trans).

EXEMPLES 12 ET 13

Chloro-5 hydroxy-3 pentyl-3 tosyl-1 indoline-2 carboxylate de méthyle: isomère cis, isomère trans.

A) Chloro-4 hexanoyl-2 N-tosyl aniline.

On chauffe à 100 ° C pendant une nuit dans 100 ml de pyridine un mélange contenant 15 g de chloro-4 hexanoyl-2 aniline et 10,5 g de chlorure de tosyle. On évapore le solvant, reprend par de l'eau chlorhydrique, extrait au chlorure de méthylène, sèche et concentre. Le brut de réaction cristallise dans l'éther isopropylique, on obtient 14,5 g.

Fc = 78-80 ° C.

B) Chloro-4 hexanoyl-2 N-méthoxycarbonylméthyl N-tosyl aniline.

14 g du composé obtenu à l'étape précédente sont traités à 0 ° C sous argon par 1 g d'hydrure de sodium dans le DMF. Après 15 minutes sous agitation, on ajoute 22,5 g de bromoacétate de méthyle et

on laisse sous agitation une nuit à température ambiante. Après évaporation du solvant et de l'excès de dérivé bromé à la pompe à vide, on reprend par de l'eau, extrait au chlorure de méthylène, sèche et concentre puis le brut de réaction est chromatographié sur silice en éluant par un mélange DCM/pentane (50/50, v/v). On obtient 12,1 g du produit attendu.

Fc = 68-70 ° C.

C) 5 g du composé obtenu à l'étape précédente sont dissous à 0 ° C dans 100 ml de méthanol et traités par 600 mg de méthylate de sodium. Après 10 minutes, on voit la disparition du produit de départ par CCM. On ajoute de la carboglace, évapore en partie le solvant, reprend par de l'eau, extrait au chlorure de méthylène, sèche et concentre. Le brut de réaction est chromatographié sur silice en éluant par DCM qui sépare les 2 isomères. L'isomère le moins polaire est recristallisé à froid dans le mélange éther-cyclohexane. On obtient 0,85 g.

Fc = 95-96 ° C : isomère cis.

L'isomère le plus polaire est recristallisé dans l'éther iso. On obtient 2 g de produit.

Fc = 102-104 ° C : isomère trans.

EXEMPLES 14 ET 15

Butylsulfonyl-1 chloro-5 (chloro-2 phényl)-3 hydroxy-3 indoline-2 carboxylate de méthyle, isomère cis, isomère trans.

A) Butylsulfonamido-2 dichloro-2′,5 benzophénone.

On agite pendant 9 jours à température ambiante 11 g de dichloro-2′,5aminobenzophénone et 8,2g de chlorure de n-butanesulfonyle dans 40 ml de pyridine. On évapore la pyridine sous vide, ajoute de l'eau, extrait par 3 volumes d'acétate d'éthyle, lave la phase organique par de l'eau chlorhydrique, de l'eau saline et sèche sur sulfate de magnésium. Après évaporation du solvant, on chromatographie sur gel de silice en éluant le produit attendu par le mélange pentane-acétate d'éthyle (90/10, v/v). On recueille 4,4 g.

B) (N-butylsulfonyl N-méthoxycarbonylméthyl) amino-2 dichloro-2′5 benzophénone.

On dissout à 0 ° C, sous argon, dans 40 ml de DMF anhydre, 4 g du composé obtenu à l'étape précédente, on traite par 320 mg d'hydrure de sodium à 80 % pendant 15 minutes puis on ajoute en 2 heures 6,5 g de bromacétate d'éthyle et on laisse 6 heures à température ambiante. On ajoute de l'eau puis on extrait le produit de la réaction et on filtre sur gel de silice en éluant par DCM. On obtient le produit attendu sous forme d'une huile épaisse.

C) 4,3 g du composé obtenu à l'étape précédente sont placés dans 50 ml de méthanol à 0 ° C et traités pendant 3 heures par 54 mg de méthylate de sodium. Après disparition du produit de départ (vérifiée en CCM), on verse dans un grand volume d'eau, extrait par 3 volumes d'acétate d'éthyle, lave la phase organique par de l'eau, de l'eau saline, sèche sur sulfate de magnésium puis évapore le solvant sous vide. Le résidu est chromatographié sur gel de silice.

Le premier isomère est élué par le DCM.

Fc = 140-143 ° C : isomère cis.

(recristallisation dans DCM-éther iso).

Le second isomère (isomère trans) est élué par le mélange DCM-éther iso.

Fc = 161-163 ° C : isomère trans.

(recristallisation dans DCM-éther iso).

EXEMPLES 16 ET 17

Chloro-5 (chloro-2 phényl)-3 (diméthoxy-2,5 phénylsulfonyl)-1 hydroxy-3 indoline-2 carboxylate de méthyle, isomère cis isomère trans.

A) Dichloro-2′,5 (diméthoxy-2,5 phénylsulfonamido)-2 benzophénone.

Ce composé est préparé selon le mode opératoire décrit dans les exemples précédents.

B) Dichloro-2′,5 (N-diméthoxy-2,5 phénylsulfonyl N-méthoxycarbonylméthyl) amino-2 benzophénone.

On dissout 8,2 g du composé préparé à l'étape précédente à 0 ° C sous argon dans 60 ml de DMF anhydre et on ajoute 550 mg d'hydrure de sodium à 80 % puis après 15 minutes 8 g de bromacétate de méthyle et on laisse sous agitation pendant 10 heures à température ambiante. On verse le milieu réactionnel dans de l'eau, filtre le solide formé puis on le dissout dans l'acétate d'éthyle. La phase organique est lavée par de l'eau, de l'eau saline, séchée sur sulfate de magnésium et évaporée sous

vide. Le solide est recristallisé du mélange DCM-éther iso.

Fc = 129-131 °C.

C) Chloro-5 (chloro-2 phényl)-3 (diméthoxy-2,5 phénylsulfonyl)-2 hydroxy-3 indoline-2 carboxylate de méthyle.

L'étape de cyclisation peut être réalisée en présence de différents réactifs.

a) On dissout à 0 °C 1 g du composé obtenu à l'étape précédente dans 10 ml de DCM, on ajoute 145 mg de DBU et on conserve le milieu réactionnel pendant 24 heures à + 5 °C. Le milieu réactionnel est versé directement sur colonne de silice. On élue par DCM.

Le composé élué le premier (231 mg) est recristallisé dans DCM-éther iso.

Fc = 168-169 °C (isomère cis).

L'isomère trans est élué ensuite.

Fc = 193-195 °C. Recristallisation DCM-éther iso.

b) On dissout 800 mg du composé obtenu à l'étape B dans 5 ml de THF anhydre, on ajoute 0,8 ml de HMPA et on porte à -78 °C sous atmosphère d'argon. On ajoute à la seringue 1 ml d'une solution du complexe LDA, TFA (1,5 M) dans le cyclohexane ; après 45 minutes, on ajoute 0,3 ml de LDA supplémentaire puis on ajoute de l'eau à -78 °C et on extrait par de l'acétate d'éthyle.

L'analyse RMN du produit obtenu montre l'existence de l'isomère cis (39 %) et de l'isomère trans (61 %).

c) On dissout 400 mg du composé obtenu à l'étape B dans 3 ml de THF anhydre sous argon, on refroidit à -78 °C puis l'on ajoute 0,9 ml d'une solution molaire de LiHMDS dans le THF puis 0,4 ml de HMPA dans 2 ml de THF. Après 60 minutes sous agitation à 78 °C, on ajoute 0,3 ml de LiHMDS supplémentaire et après 10 minutes, on ajoute de l'eau à -78 °C puis on extrait à l'acétate d'éthyle.

L'analyse RMN du produit obtenu montre l'existence de l'isomère cis (60 %) et de l'isomère trans (40 %).

d) On dissout 400 mg du composé obtenu à l'étape B dans 3 ml de THF anhydre, on refroidit à -78 °C sous argon puis l'on ajoute 1,8 ml d'une solution de KHMDS (0,5 M) dans 1 ml de toluène et 0,4 ml de HMPA dans 1 ml de THF. La solution obtenue est maintenue à -78 °C pendant 20 minutes puis on ajoute de l'eau et extrait à l'acétate d'éthyle.

L'analyse RMN du produit obtenu montre l'existence de l'isomère cis (32 %) et de l'isomère trans (68 %).

e) On refroidit à -70 °C sous argon 2 ml de THF et 0,4 ml de HMPA et l'on ajoute LiHMDS en solution molaire dans 0,9 ml de THF ; puis goutte à goutte 400 mg du composé préparé à l'étape B dans 3 ml de THF.

Après une heure à -70 °C, on ajoute de l'eau puis on extrait à l'acétate d'éthyle. L'analyse RMN du produit obtenu montre l'existence de l'isomère cis (63 %) et de l'isomère trans (37 %).

f) On dissout 1 g de diphényl-1,3 tétraméthyl-1,1,3,3 disilazane dans 4 ml de THF anhydre et l'on ajoute à -10 °C, sous argon, 2,2 ml d'une solution 1,6 M de butyl lithium dans l'hexane et 1,8 ml de THF.

Parallèlement, on dissout 400 mg du composé préparé à l'étape B dans 3 ml de THF anhydre, à -78 °C sous argon et l'on ajoute en 5 minutes 2,2 ml de la solution précédemment préparée puis 0,4 ml de HMPA. Après 1 heure, on ajoute à nouveau 0,5 ml de la solution préalablement préparée puis on laisse 1 heure à -78 °C enfin, on ajoute de l'eau et extrait à l'éther.

L'analyse RMN du produit obtenu montre l'existence de l'isomère cis(57 %) et de l'isomère trans (43 %).

EXEMPLES 18, 19 ET 20

Chloro-5 cyclohexyl-3 hydroxy-3 tosyl-1 indoline-2 carboxylate d'isopentyle, isomère trans, isomère cis, et chloro-5 cyclohexyl-3 triméthylsilyloxy-3 tosyl-1 indoline-2 carboxylate d'isopentyle, isomère cis.

A) Chloro-5 tosylamino-2 phénylcyclohexylcétone.

Ce composé est préparé selon le mode opératoire décrit dans les exemples précédents.

B) (N-tosyl N-isopentyloxycarbonylméthyl) amino-2 chloro-5 phénylcyclohexylcétone.

On dissout 5,7 g du composé préparé à l'étape précédente dans 50 ml de DMF anhydre, on ajoute 420 mg d'hydrure de sodium à 80 % puis, après 15 minutes, 12 g de bromacétate d'isopentyle et on laisse sous agitation pendant 8 heures à température ambiante. Après extraction, on chromatographie sur gel de silice et on élue une huile par les mélanges pentane-DCM (20/80, v/v) à DCM pur.

C) Chloro-5 cyclohexyl-3 hydroxy-3 tosyl-1 indoline-2 carboxylate d'isopentyle, isomère trans.

On dissout 1,6 g du composé obtenu à l'étape précédente dans 10 ml de méthyl-3 butanol anhydre. On refroidit à 0 ° C et l'on ajoute 7 mg de méthylate de sodium puis on ramène à température ordinaire en 150 minutes. On ajoute de la neige carbonique, de l'eau, extrait par décantation, lave par de l'eau, de l'eau saline et sèche sur sulfate de magnésium. On évapore le solvant sous vide puis chromatographie sur gel de silice. On élue par DCM un mélange de 2 isomères (1,6 g), on recristallise dans le mélange DCM-éther iso : SR 47275 (isomère trans).

D) Chloro-5 cyclohexyl-3 triméthylsilyloxy-3 tosyl-1 indoline-2 carboxylate d'isopentyle, isomère cis.

Les eaux mères de cristallisation de l'isomère trans (980 mg) sont dissoutes sous argon dans 8 ml d'hexaméthyldisilazane en présence de 100 mg d'imidazole et on chauffe à 120 ° C. La réaction est suivie par CCM sur gel de silice en éluant par DCM.

Après 1 heure apparait un produit peu polaire, et après une nuit un deuxième composé un peu plus polaire que le précédent apparait. On évapore à siccité sous vide et chromatographie le résidu sur gel de silice. Le composé le moins polaire est élué par le mélange DCM-pentane (50/50, v/v). On recueille 304 mg qui sont recristallisés dans le mélange DCM-éther iso.

Fc = 118-121 ° C.

E) Chloro-5 cyclohexyl-3 hydroxy-3 tosyl-1 indoline-2 carboxylate d'isopentyle, isomère cis.

A une suspension des eaux-mères de l'isomère cis isolé précédemment, (220 mg) dans 1 ml d'eau et 3 ml de THF, on ajoute 2,4 mg de-soude solide. Après 3 heures on ajoute de l'eau , évapore une partie du THF et extrait sous vide à température ambiante selon les méthodes habituelles. Le résidu est chromatographié sur silice en éluant par un mélange DCM-pentane (95/5, v/v). Le composé obtenu est sous forme cis à 87 %, d'après le spectre RMN.

EXEMPLE 21

Chloro-5 cyclohexyl-3 hydroxy-3 tosyl-1 indoline-2 carboxylate de butyle.

Ce composé est préparé par action du bromacétate de butyle sur le chloro-5 tosylamino-2 phénylcyclohexylcétone puis cyclisation en présence de méthylate de sodium dans le butanol.

Le composé formé est l'isomère trans.

Fc = 115 ° C.

EXEMPLES 22 ET 23

Chloro-5 (chloro-2 phényl)-3 hydroxy-3 méthyl-2 tosyl-1 indoline-2 carboxylate de méthyle, isomère cis, isomère trans.

Un mélange contenant 0,5 g de dichloro-2′,5 [N-(méthoxycarbonyl-1éthyl) N-tosyl] amino-2 benzophénone, 0,1 g de méthylate de sodium et 2 ml de DMF est placé sous agitation pendant 20 heures à température ambiante sous azote. On concentre sous vide, reprend par de l'eau, essore le précipité et lave à l'eau. Le résidu est chromatographié sur silice en éluant par DCM. On obtient 60 mg d'isomère cis et 250 mg d'isomère trans.

EXEMPLE 24

Chloro-5 (chloro-2 phényl)-3 (cyano-4 phénylsulfonyl)-1 hydroxy-3 indoline-2 carboxylate de méthyle, isomère cis.

A) [N-(cyano-4 phénylsulfonyl)] amino-2 dichloro-2',5 benzophénone.

On chauffe à 100 ° C pendant 48 heures dans la pyridine, 10 g d'amino-2 dichloro-2'5 benzophénone et 7,7 g de chlorure de cyano-4 phénylsulfonyle en présence de 4,6 g de DMAP, on évapore à siccité, ajoute de l'eau et de l'acétate d'éthyle, lave la phase organique par de l'eau chlorhydrique diluée, de l'eau, de l'eau saline, sèche sur sulfate de magnésium et évapore le solvant sous vide. Le précipité formé est filtré puis on recristallise 2 fois dans le mélange DCM-éther iso pour obtenir le produit attendu

Fc = 172-173 ° C.

B) [N-(cyano-4 phénylsulfonyl) N-méthoxycarbonylméthyl] amino-2 dichloro-2',5 benzophénone.

On dissout à 0 ° C, dans 70 ml de DMF, sous argon, 10 g du composé obtenu à l'étape précédente puis on ajoute 740 mg d'hydrure de sodium à 80 %, et après 15 minutes 14 g de bromacétate de méthyle. Après 24 heures, on ajoute de l'eau, décante la phase aqueuse, extrait le solide obtenu par

AcOEt, lave la phase organique par de l'eau, de l'eau saline, sèche sur sulfate de magnésium et évapore sous vide. On obtient le produit attendu qui est recristallisé dans DCM-éther iso.

Fc = 186-188 ° C.

C) 2 g du composé précédent sont mis en suspension à 0 ° C dans 40 ml d'un mélange MeOH/THF (1/1, v/v) et traités par 100 mg de méthylate de sodium. Après 3 heures à température ordinaire, on observe une dissolution totale. On évapore partiellement les solvants sous vide, ajoute une grande quantité d'eau et extrait à l'acétate d'éthyle. On lave à l'eau, l'eau saline, sèche sur sulfate de magnésium et évapore sous vide. Le résidu est chromatographié sur gel de silice. Le chlorure de méthylène élue successivement les 2 isomères.

L'isomère le moins polaire est recristallisé dans DCM-éther iso

Fc = 222-223 ° C (isomère cis).

EXEMPLES 25 ET 26

Chloro-5 (chloro-2 phényl)-3 (diméthoxy-3,4 phénylsulfonyl)-1 hydroxy-3 indoline-2 carboxylate de méthyle, isomère trans, isomère cis.

A) Dichloro-2′,5 (diméthoxy-3,4 phénylsulfonamido)-2 benzophénone.

On chauffe dans la pyridine pendant une nuit à 100 ° C, 5,6 g d'amino-2 dichloro-2',5 benzophénone et 5 g de chlorure de diméthoxy-3,4 phénylsulfonyle. On évapore la pyridine à siccité, ajoute de l'eau, de l'acétate d'éthyle contenant un peu de DCM et extrait.Après lavages à l'eau et séchage sur sulfate de sodium on évapore sous vide et recristallise 7,7 g du produit attendu dans le mélange DCM-AcOEt.

Fc = 164 ° C.

B) Dichloro-2′,5 (N-diméthoxy-3,4 phénylsulfonyl N-méthoxycarbonylméthyl) amino-2 benzophénone.

On dissout 7,2 g du composé obtenu à l'étape précédente, à 0 ° C sous azote, dans du DMF anhydre. On ajoute 500 mg d'hydrure de sodium et après 10 minutes 9,5 g de bromacétate d'éthyle. Après 1 nuit, on ajoute un excès d'eau et filtre le précipité obtenu. On dissout dans DCM, sèche sur sulfate de magnésium et évapore le solvant. 7,7 g du produit attendu sont recristallisés dans le mélange DCM-éther iso.

Fc = 164 ° C.

C) Isomère trans.

Une suspension de 7 g du produit obtenu à l'étape précédente est refroidie à 0 ° C sous azote dans 90 ml de méthanol contenant 2 ml de THF et traitée par 720 mg de méthylate de sodium. Après 2 heures à température ordinaire, on filtre le produit de départ qui n'a pas réagi, ajoute un grand volume d'eau, de la carboglace et extrait par l'acétate d'éthyle. Il apparait 4 produits en CCM. Le produit le plus abondant est recristallisé 2 fois dans le mélange DCM-éther iso.

Fc = 184-185 ° C : isomère trans.

D) Isomère cis.

On dissout à 0 ° C dans 13 ml de DCM 1,3 g du composé obtenu à l'étape B, en présence de 180 mg de DBU. Après une nuit sous agitation, on verse directement le milieu réactionnel sur une colonne de silice préparée dans DCM et l'on sépare ainsi par DCM éluant le mélange des composés de cyclisation. On effectue ensuite une chromatographie sur alumine en éluant par le mélange DCM-éther iso (70/30 ; v/v). On isole ainsi l'isomère cis.

Spectre de RMN à 200 MHz dans le DMSO, à T 370 °K.

| Delta | Aspect | Intégration | Attribution |
|---|---|---|---|
| 2,50 | | | DMSO |
| 3,15 | s | 3 H | $CO_2CH_3$ |
| 3,80 | s | 3 H | $OCH_3$ |
| 3,85 | s | 3 H | $OCH_3$ |
| 5,10 | s | 1 H | CH |
| 6,50 | s | 1 H | OH |
| 7,00 à 7,80 | m | 10 H | H aromatiques |

EXEMPLES 27, 28, 29 ET 30

Chloro-5 (chloro-2 phényl)-3 hydroxy-3 p-tosyl-1 indoline-2 carboxylate de benzyle, isomère trans, isomère cis

et acide chloro-5 (chloro-2 phényl)-3 hydroxy-3 p-tosyl-1 indoline-2 carboxylique,isomère trans et isomère cis.

A) Dichloro-2',5 (N-tosyl N-benzyloxycarbonylméthyl) amino-2 benzophénone.

On fait réagir 20 g de dichloro-2',5 N-tosylamino-2 benzophénone (préparé selon la méthode habituelle) et 1,6 g d'hydrure de sodium dans 100 ml de DMF. Après 15 minutes d'agitation, on ajoute 34 g de bromacétate de benzyle et on laisse une nuit à température ambiante. On évapore le DMF, reprend par de l'eau, extrait au chlorure de méthylène, sèche et concentre. Le brut obtenu est chromatographié sur gel de silice en éluant par DCM. Le produit obtenu 14,39 g est recristallisé dans l'éther iso.

Fc = 99-101 °C.

B) Chloro-5 (chloro-2 phényl)-3 hydroxy-3 p-tosyl-1 indoline-2 carboxylate de benzyle, isomère trans.

1 g du composé obtenu à l'étape précédente est traité à -78 °C dans 1,2 ml de LDA concentré à 1,5 M dans la cyclohexane. Après 3 heures, on reprend par de l'eau, extrait par DCM, sèche et concentre. Le brut de réaction est chromatographié sur gel de silice en éluant par DCM et on sépare le mélange des 2 isomères. L'isomère le plus polaire est recristallisé 2 fois dans le mélange DCM-éther iso. On obtient 600 mg d'isomère trans.

Fc = 168-169 °C (recristallisation DCM-éther iso)

C) Chloro-5 (chloro-2 phényl)-3 hydroxy-3 p-tosyl-1 indoline-2 carboxylate de benzyle, isomère cis.

2 g du composé préparé à l'étape A sont mis en solution à 0 °C dans DCM et l'on ajoute 540 mg de DBU. Après 20 minutes à 0 °C, on ajoute une solution de sulfate de potassium, de l'eau, puis on extrait, sèche et concentre. Par chromatographie sur silice en éluant par DCM on obtient 450 mg du produit le moins polaire (isomère cis) et 700 mg du produit le plus polaire (isomère trans).

L'isomère cis est obtenu sous forme de mousse

Analyse élémentaire

| | | | | |
|---|---|---|---|---|
| - | calculé % | C : 61,27 | H : 4,05 | N : 2,46 |
| - | trouvé % | 61,29 | 4,20 | 2,48 |

D) Acide chloro-5 (chloro-2 phényl)-3 hydroxy-3 p-tosyl-1 indoline-2 carboxylique, isomère trans.

On dissout 500 mg de l'isomère trans du composé préparé à l'étape B dans 300 ml d'acétate d'éthyle en présence de 100 mg de Palladium sur charbon. Après 10 mn d'hydrogenolyse le produit cristallise. On filtre le palladium puis on solubilise les cristaux avec du DMF chaud. On concentre le DMF, reprend par un grand volume de THF. On concentre, ajoute du méthanol, le produit cristallise. On concentre 175 mg du produit attendu (isomère trans).

RMN

| Delta | Aspect | Intégration | Attribution |
|---|---|---|---|
| 2,44 | s | 3 H | CH3 (tosyle) |
| 4,87 | s | 1 H | H6 (chloro-2 phényle) |
| 6,74 | d | 1 H | H4 (indole) |
| 6,98 | s | 1 H | OH |
| 7,28 - 7,53 | m | 7 H | H aromatiques |
| 7,88 | d | | OH |
| | (J=8,6Hz) | 2 H | H2,H6 (tosyle) |

De la même façon, l'isomère cis de l'acide est préparé par hydrogénolyse de l'ester benzylique obtenu à l'étape C.

Fc = 209-212 ° C.

EXEMPLE 31

Acide chloro-5 (chloro-2 phényl)-3 (diméthoxy-3,4 phénylsulfonyl)-1 hydroxy-3 indoline-2 carboxylique, isomère cis.

Cet acide est préparé selon le mode opératoire décrit dans l'exemple précédent, via l'ester benzylique dudit acide.

Fc = 130-132 ° C.

Des esters méthyliques de formule (I) ont été préparés selon des modes opératoires analogues. Ils sont décrits dans le tableau 1 ci-dessous.

TABLEAU 1

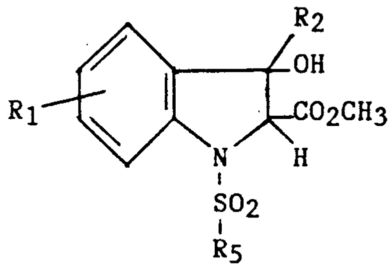

(I)

Pour chaque composé de formule (I) ayant les substituants $R_1$, $R_2$ et $R_5$ du tableau ci-dessous, l'isomère cis est indiqué, puis l'isomère trans, sauf indication contraire.

| Exemple | $R_1$ | $R_2$ | $R_5$ | F °C Solvant |
|---|---|---|---|---|
| 32 | H | phényle | p-tolyle | 154 |
| 33 | | | | 170 |
| 34 | Cl-5 | cyclopentyle | p-tolyle | 187-190<br>DCM-MeOH |
| 35 | | | | 153-157<br>DCM-éther iso |
| 36 | Cl-5 | cyclohexyle | p-tolyle | 180<br>éther-cyclohexane |
| 37 | | | | 144<br>éther-cyclohexane |
| 38 | Cl-5 | cyclohexyle | naphtyl-2 | 177<br>MeOH |
| 39 | | | | 150 |

| | | | | | |
|---|---|---|---|---|---|
| | | | | éther-cyclohexane |
| 40 | Cl-5 | isopropyle | p-tolyle | 158<br>DCM-éther iso |
| 41 | | | | 172<br>DCM-éther iso |
| 42 | Cl-5 | F-2 phényle | p-tolyle | 165-166<br>DCM-éther iso |
| 43 | | | | 212-213<br>DCM-éther iso |
| 44<br>(*) | Cl-5 | phényle | p-tolyle | 206<br>DCM-éther iso-MeOH |
| 45 | | | | 193-194<br>AcOEt - MeOH |
| 46 | Cl-5 | cycloheptyle | p-tolyle | 170-172<br>éther iso |
| 47 | | | | 154-155 |
| 48 | Cl-5 | Cl-2 phényle | p-tolyle | 174 |
| 49 | | | | 255 |
| 50 | Cl-5 | cyclohexyle | diméthylamino-4-phényle | 184-185 |
| 51 | | | | 198-200<br>DCM-éther iso |

| | | | | | |
|---|---|---|---|---|---|
| 52 | Cl-5 | cyclohexyle | triméthyl-2,4,6 phényle | 139-142 DCM-éther iso | |
| 53 | | | | 200-203 DCM-éther iso | |
| 54 | Cl-5 | cyclohexyle | n-butyle | 150-153 MeOH-éther iso | |
| 55 | Cl-5 | Cl-2 phényle | $CF_3$-2 phényle | 216 | |
| 56 | | | $CF_3$-2 phényle | 242 | |
| 57 | Cl-5 | Cl-2 phényle | benzyloxy-4 phényle | 166 DCM-éther iso | |
| 58 | | | | 195 DCM-éther iso | |
| 59 | Cl-5 | Cl-2 phényle | Cl-4 phényle | 174 | |
| 60 | | | | 230 | |
| 61 | Cl-5 | Cl-4 phényle | p-tolyle | 224 | |
| 62 | | | | 186 EtOH | |
| 63 | Cl-5 | $CH_3$-2 phényle | p-tolyle | 168 | |
| 64 | | | | 238 AcOEt | |

| | | | | |
|---|---|---|---|---|
| 65 | Cl-5 | Cl-2 phényle | OH-4 phényle | RMN (**) |
| 66 | | | | 163<br>MeOH-éther iso |
| 67 | Cl-5 | Cl-2 phényle | Cl-3 phényle | 175 |
| 68 | | | | 186 |
| 69 | Cl-5 | Cl-2 phényle | m-tolyle | 173 |
| 70 | | | | 229 |
| 71 | Cl-5 | méthoxy-2 phényle | p-tolyle | 165 |
| 72 | | | | 240 |
| 73 | Cl-5 | Cl-3 phényle | p-tolyle | 137 |
| 74 | | | | 210 |
| 75 | Cl-5 | méthyl-2 phényle | diCl-3,4 phényle | 196 |
| 76 | | | | 175 |

| | | | | |
|---|---|---|---|---|
| 77 cis | Cl-5 | Cl-2 phényle | méthoxy-3 phényle | 132 |
| 78 | Cl-5 | Cl-2 phényle | triméthoxy-2,3,4 phényle | 207 |
| 79 | | | | 183 |
| 80 | Cl-5 | Cl-2 phényle | butoxy-4 phényle | 124-125 hexane |
| 81 | | | | 190-192 MeOH-éther iso |
| 82 | Cl-5 | Cl-2 phényle | trifluorométhoxy-4 phényle | 170 |
| 83 | | | | 166 |
| 84 | Br-5 | F-2 phényle | diméthoxy-3,4 phényle | 162-164 |
| 85 | | | | 162-165 DCM-éther iso |
| 86 | Cl-5 | Cl-2 phényle | phényle | 148 éther iso |
| 87 | | | | 230 DCM-éther iso |
| 88 | Cl-5 | Cl-2 phényle | méthoxy-4 phényle | 173 hexane-éther iso |
| 89 | | | | 217 hexane-éther iso |
| 90 | Br-5 | Cl-2 phényle | diméthoxy-3,4 phényle | 160-162 |
| 91 | | | | 199 |

| | | | | | |
|---|---|---|---|---|---|
| 92 | Cl-5 | Cl-2 phényle | éthoxy-4 phényle | 174 hexane-éther iso | |
| 93 | | | | 186 hexane-éther iso | |
| 94 trans | $CH_3O$ -2 | Cl-2 | p-tolyle | 215 | |
| 95 | $CH_3$-5 | Cl-2 phényle | p-tolyle | 165 | |
| 96 | | | | 216 | EtOH |
| 97 | $CF_3$-5 | $CF_3$-2 phényle | p-tolyle | 189 | |
| 98 | | | | 202 | |
| 99 | Cl-5 | $CF_3$-2 phényle | p-tolyle | 166 | |
| 100 | | | | 205 | |
| 101 trans | Cl-5 | Cl-2 phényle | méthoxy-3 phényle | 206,5 | |
| 102 | Cl-5 | Cl-2 phényle | $CF_3$-4 phényle | 191 | |
| 103 | | | | 180 | |

* Pour ce composé, le dérivé silylé (VII) a été préparé : isomère <u>cis</u> Fc = 176-178°C.

(**) Exemple 65 : Spectre RMN à 200 MHz, dans le DMSO, à T = 380°K.

| Delta | Aspect | Intégration | Attribution |
|---|---|---|---|
| 2,45 | | | DMSO |
| 3,10 | s | 3 H | $CO_2CH_3$ |
| 5,00 | s | 1 H | CH |
| 6,30 | s | 1 H | OH |
| 6,80-7,80 | m | 11 H | aromatiques |

EXEMPLES 104 ET 105

N,N-diméthyl chloro-5 cyclohexyl-3 hydroxy-3 tosyl-1 indoline-2 carboxamide : isomère trans et isomère cis.

A) chloro-5 tosylamino-2 phénylcyclohexylcétone.

Ce composé est celui qui a été préparé à l'exemple 18, étape A.

B) Bromure de diméthylacétamide.

On refroidit à 0°C une solution contenant 56 g de bromure de bromacétyle dans 100 ml de DCM, et on fait barboter de la diméthylamine gazeuse jusqu'à basicité du milieu. On filtre, sèche et concentre l'amide brute obtenue sous forme huileuse (22 g).

C) (N-tosyl N-diméthylcarbamoylméthyl) amino-2 chloro-5 phénylcyclohexylcétone.

4,3 g de chloro-5 tosylamino-2 phénylcyclohexylcétone sont placés dans 20 ml de DMF en présence de 360 mg d'hydrure de sodium à 80 %, après 15 minutes à TA, on ajoute 5,4 g du composé préparé à l'étape B et on laisse sous agitation pendant une nuit à TA. On verse le milieu réactionnel sur de l'eau, filtre le précipité, puis on reprend le précipité par du DCM, sèche et concentre. Le produit attendu cristallise dans l'éther isopropylique.

m = 3,9 g

Fc = 184-187°C.

D) N,N-diméthyl chloro-5 cyclohexyl-3 hydroxy-3 tosyl-1 indoline-2 carboxamide : isomère trans.

1,5 g du composé obtenu à l'étape précédente est porté à -78°C dans 20 ml de THF anhydre, on ajoute 2,3 ml de LDA en solution 1,5 M dans le cyclohexane et on laisse 2 heures sous agitation à -78°C. On verse sur l'eau, extrait au DCM, sèche et concentre. Le produit brut obtenu est chromatographié sur silice, le mélange AcOEt/DCM (10/90 ; v/v) élue un composé dont la RMN montre qu'il est l'isomère trans. On le recristallise dans un mélange éther isopropylique/DCM.

Fc = 179-182°C.

E) N,N-diméthyl chloro-5 cyclohexyl-3 hydroxy-3 tosyl-1 indoline-2 carboxamide : isomère cis.

1,5 g du produit obtenu à l'étape C est traité par 950 mg de DBU dans 10 ml de DCM pendant 24 heures à TA. On effectue ensuite une chromatographie sur silice du milieu réactionnel en éluant par le mélange DCM/AcOEt (95/5 ; v/v) pour obtenir l'autre isomère (isomère cis). On recristallise dans l'éther isopropylique.

m = 120 mg

Fc = 152-155°C.

EXEMPLES 106 ET 107

N,N-diméthyl chloro-5 (chloro-2 phényl)-3 hydroxy-3 tosyl-1 indoline-2 carboxamide : isomère trans et isomère cis.

A) dichloro-5,2′ tosylamino-2 benzophénone.

Ce composé est préparé selon la méthode habituelle.

B) (N-tosyl N-diméthylcarbamoylyl) amino-2 dichloro-5,2′ benzophénone.

8,4 g de dichloro-5,2′ tosylamino-2 benzophénone sont mis en solution dans 40 ml de DMF et traités par 0,7 g d'hydrure de sodium à 80 % ; après 15 minutes, on ajoute 10 g de bromure de diméthylacétamide, préparé à l'exemple 1 et on laisse une nuit sous agitation à TA. On verse le milieu réactionnel sur de l'eau, filtre le précipité formé, reprend par du DCM, sèche et concentre. Après cristallisation dans l'éther iso, on obtient 9,2 g du produit attendu.

Fc = 154-157 °C.

C) N,N-diméthyl chloro-5 (chloro-2 phényl)-3 hydroxy-3 tosyl-1 indoline-2 carboxamide : isomère trans.

1,5 g du composé obtenu à l'étape précédente est traité à -78 °C dans 100 ml de THF anhydre par 2,6 ml de solution 1,5 M de LDA dans le cyclohexane pendant 3 heures. On verse sur de l'eau, extrait au DCM, sèche et concentre. On chromatographie sur silice en éluant par un mélange DCM/AcOEt (94/6 ; v/v). On recueille un peu de composé moins polaire puis le composé le plus polaire est élué : l'isomère trans.

On le recristallise dans un mélange DCM/éther iso.

Fc = 232-233 °C.

D) N,N-diméthyl chloro-5 (chloro-2 phényl)-3 hydroxy-3 tosyl-1 indoline-2 carboxamide : isomère cis.

1,5 g du composé préparé à l'étape B est porté à reflux du DCM pendant 24 heures, en présence de 900 mg de DBU. On chromatographie sur colonne de silice en éluant par un mélange DCM/AcOEt (95/5 ; v/v). Après recristallisation dans l'éther iso, on obtient 190 mg du produit attendu.

Fc = 220-221 °C.

E) On dissout le composé de l'étape B (1,0 g) dans l'acétonitrile (25 ml) et ajoute 109 mg de soude dans 2 ml d'eau. Le milieu est hétérogène, on l'agite à 45 °C pendant 1 heure violemment grâce à une turbine et à l'air comprimé, soit en présence de 110 mg de chlorure de benzyltriéthylammonium, soit sans l'ajout de ce réactif.

Après extraction, on dose le produit obtenu sous forme d'un mélange des 2 isomères cis et trans. Le rapport des 2 isomères observés par RMN à 360 °K dans le DMSO est 1/1.

EXEMPLE 108

N,N-diméthyl (allyloxy-4 phénylsulfonyl)-1 chloro-5 (chloro-2 phényl)-3 hydroxy-3 indoline-2 carboxamide : isomère cis.

A) Allyloxy-4 phénylsulfonate de sodium.

On dissout dans 60 ml d'éthanol et 50 ml de soude à 15 %, 30 g de phénylsulfonate de sodium, on ajoute 20 g de bromure d'allyle et on porte à reflux pendant 48 heures. On concentre l'éthanol et filtre le précipité obtenu, puis on sèche le précipité sous vide en présence d'anhydride phosphorique. On obtient 23,3 g du produit attendu.

B) Chlorure d'allyloxy-4 phénylsulfonyle.

Le produit obtenu à l'étape précédente (23,3 g) est traité par 24 g de pentachlorophosphore pendant une nuit à reflux dans 300 ml de DCM. On filtre le milieu, concentre et l'on obtient une huile (16,5 g).

C) (N-allyloxy-4 phénylsulfonylamido)-2 dichloro-2′,5 benzophénone.

On ajoute le chlorure de sulfonyle obtenu à l'étape précédente à 19 g de dichloro-2′,5 amino-2 benzophénone dans 200 ml de pyridine. Après une nuit à température ambiante, on concentre la pyridine, reprend par de l'eau chlorhydrique, extrait au DCM, sèche et concentre. Le brut est chromatographié sur silice, le mélange DCM/pentane (50/50 ; v/v) élue le produit attendu que l'on cristallise dans le mélange DCM/éther iso. On obtient 8,5 g du produit attendu.

Fc = 96-97 °C.

D) [(N-allyloxy-4 phénylsulfonyl) N-(N′,N′-diméthylcarbamoylméthyl)] amino-2 dichloro-2',5 benzophénone.

4 g du produit obtenu à l'étape précédente sont dissous dans 20 ml de DMF sous azote, on ajoute 310 mg d'hydrure de sodium à 80 %, agite pendant 15 minutes à TA, puis ajoute 3,1 g de bromo N,N-diméthylacétamide. Après une nuit à TA, on verse le milieu sur de l'eau, filtre le produit, reprend par du DCM, sèche et concentre puis on cristallise dans le mélange DCM/éther iso pour obtenir 4 g du produit attendu, enfin on recristallise dans le même mélange de solvants :

Fc = 133-135 °C.

E) N,N-diméthyl (allyloxy-4 phénylsulfonyl)-1 chloro-5 (chloro-2 phényl)-3 hydroxy-3 indoline-2 carboxamide, isomère cis.

3,8 g du produit obtenu à l'étape précédente sont traités à 30 °C par 1,8 g de DBU dans 20 ml de DCM pendant 3 jours. Le milieu est chromatographié sur alumine, le DCM élue le composé le moins polaire que l'on recristallise du mélange DCM/éther iso. On obtient m = 840 mg

Fc = 181-182 °C.

EXEMPLE 109

N-benzyl N-méthyl chloro-5 (chloro-2 phényl)-3 hydroxy-3 (diméthoxy-3,4 phénylsulfonyl)-1 indoline-2 carboxamide, isomère cis.

A) N-méthyl N-benzyl bromacétamide.

A 0 °C, on ajoute 10 g de bromure de bromacétyle en solution dans 20 ml de DCM à une solution de 6 g de méthyl benzylamine et 5 g de triéthylamine dans 50 ml de DCM. Après une nuit à TA,on ajoute de l'éther, filtre le précipité formé, concentre et on recueille 12 g de produit attendu brut.

B) Dichloro-2',5 (diméthoxy-3,4 phénylsulfonamido)-2 benzophénone.

Ce composé a été préparé à l'exemple 25, étape A.

C) Dichloro-2',5 [(N-diméthoxy-3,4 phénylsulfonyl) N-(N'-méthyl N'-benzyl carbamoylméthyl] amino-2 benzophénone.

5,5 g du composé décrit à l'étape B sont mis en solution dans 30 ml de DMF, et traités par 400 mg d'hydrure de sodium. Après 15 minutes, on ajoute 12 g du dérivé bromé préparé à l'étape A et on laisse sous agitation à TA pendant 24 heures. On évapore le DMF, reprend par de l'eau, extrait par DCM, sèche et concentre. Le produit brut est chromatographié sur silice, le produit attendu est élué par DCM.

m = 2,1 g

Fc = 148-150 °C.

D) N-benzyl N-méthyl chloro-5 (chloro-2 phényl)-3 hydroxy-3 (diméthoxy-3,4 phénylsulfonyl)-1 indoline-2 carboxamide, isomère cis.

On traite les 2,1 g de produit obtenus à l'étape précédente par 1 g de DBU dans 20 ml de DCM pendant 3 jours. On verse le milieu réactionnel sur une colonne d'alumine, le DCM élue l'isomère le moins polaire (870 mg) sous forme huileuse. Après séchage, on obtient une mousse caractérisée par RMN.

2,81 ppm : s : 3H : $N\text{-}CH_3$

3,60 ppm : m : 8H : $2OCH_3$ + $N\text{-}CH_2\text{-}C_6H_5$

5,35 ppm : s : 1H : C$\underline{H}$ (indoline-2)

6,20-7,80 ppm : m : 16H : aromatiques + OH

EXEMPLES 110 ET 111

chloro-5 (chloro-2 phényl)-3 (diméthoxy-3,4 phénylsulfonyl)-1 hydroxy-3 pyrrolidinocarbonyl-2 indoline, isomère cis et isomère trans.

Ce composé est préparé selon le mode opératoire habituel par action du bromacétamide de pyrrolidine sur le (diméthoxy-3,4 phénylsulfonamide)-2 dichloro-5,2' benzophénone, puis, cyclisation du produit obtenu par le DBU dans le chloroforme. Un produit est élué sur une colonne d'alumine par DCM/AcOEt (90/10 ; v/v), il s'agit de l'isomère cis.

Fc = 237 °C

AcOEt élue l'isomère trans que l'on recristallise ensuite dans AcOEt.

Fc = 230 °C

EXEMPLE 112

chloro-5 (chloro-2 phényl)-3 (diméthoxy-3,4 phénylsulfonyl)-1 hydroxy-3 indoline-2 carboxamide, isomère trans.

A) dichloro-2',5 (diméthoxy-3,4 phénylsulfonamido)-2 benzophénone.

On mélange dans 300 ml de pyridine 114 g d'amino-2 dichloro-5,2' benzophénone et 100 g de chlorure de dimétoxy-3,4 phénylsulfonyle. Après 4 jours à TA, on évapore l'excès de pyridine, on reprend par de l'eau chlorhydrique, on extrait au DCM, sèche et concentre. Le produit attendu cristallise ensuite du mélange DCM/éther iso.

m = 181 g

Fc = 159-161 ° C

B) dichloro-2',5 [N-(diméthoxy-3,4 phénylsulfonyl) N-benzyloxycarbonylméthyl] amino-2 benzophénone.

172 g du produit préparé précédemment est dissous dans 800 ml de DCM et refroidi à 0 ° C. On ajoute progressivement sous azote 11,7 g d'hydrure de sodium à 80 % puis après 30 minutes, on ajoute 256 g de bromoacétate de benzyle et on laisse 24 heures sous agitation à TA. On évapore le DMF, reprend par de l'eau, extrait au DCM, sèche et concentre. Le produit attendu cristallise dans l'éther iso puis on le recristallise dans le mélange DCM/éther iso.

m = 136,5 g

Fc = 102-104 ° C

C) chloro-5 (chloro-2 phényl)-3 (diméthoxy-3,4 phénylsulfonyl)-1 hydroxy-3 indoline-2 carboxylate de benzyle, isomère trans.

3 g du produit obtenu à l'étape précédente sont traités par 740 mg de TBD dans 20 ml de DCM à TA pendant 1 heure. Le milieu réactionnel est chromatographié sur silice, le DCM élue le composé attendu sous forme d'isomère trans qui est le seul isomère formé par la réaction de cyclisation.

Le composé obtenu est recristallisé dans le mélange DCM/éther iso.

m = 2 g

Fc = 190-192 ° C

D) Acide chloro-5 (chloro-2 phényl)-3 (diméthoxy-3,4 phénylsulfonyl)-1 hydroxy-3 indoline-2 carboxylique, isomère trans.

L'ester benzylique obtenu à l'étape précédente, dans 50 ml d'AcOEt, est traité par l'hydrogène, pendant 30 minutes en présence de 100 mg de Pd/C à 10 %. On filtre le milieu réactionnel sur célite[R], lave au méthanol chaud et concentre. Le produit attendu cristallise dans le mélange DCM/éther iso.

m = 1,5 g

Fc = 218-221 ° C

E) chloro-5 (chloro-2 phényl)-3 (diméthoxy-3,4 phénylsulfonyl)-1 hydroxy-3 indoline-2 carboxamide, isomère trans.

180 mg du composé obtenu à l'étape précédente, dans 15 ml de DCM, sont traités par 140 mg de BOP en présence d'une quantité suffisante de DIPEA pour solubiliser l'acide. Après 15 minutes, on introduit de l'ammoniac gazeux pendant 10 minutes. On verse le milieu dans une solution saturée d'hydrogénocarbonate de sodium, on extrait, sèche puis concentre. Le produit brut est chromatographié sur silice, en éluant par le mélange DCM/AcOEt (80/20 ; v/v) on obtient le composé attendu que l'on recristallise ensuite du mélange DCM/éther iso.

m = 63 mg

Fc = 183-185 ° C

EXEMPLES 113 ET 114

N-isopentyl chloro-5 cyclohexyl-3 hydroxy-3 tosyl-1 indoline-2 carboxamide, isomère cis et isomère trans.

A) On prépare la [(N-tosyl) amino-2 chloro-5] phénylcyclohexylcétone selon la méthode habituelle.

B) [N-(N'-isopentyl) carbamoylméthyl N-tosyl] amino-2 chloro-5 phényl cyclohexylcétone.

7,2 g du composé obtenu précédemment dans 130 ml de DMF sont refroidis à 0 ° C et placés sous Argon, on ajoute 1,1 équivalent d'hydrure de sodium et on laisse revenir à TA. On ajoute ensuite 15,2 g de N-isopentyl bromo-2 acétamide et on laisse une nuit sous agitation à TA. On évapore le DMF, reprend par de l'eau, extrait au DCM, sèche et concentre. Le produit brut est chromatographié sur silice en éluant par le mélange éther/pentane (70/30 ; v/v).

C) N-isopentyl chloro-5 cyclohexyl-3 hydroxy-3 tosyl-1 indoline-2 carboxamide, mélange d'isomères.

A 3 g du produit précédent, dans 50 ml de THF anhydre à -20°C, on ajoute 2,1 équivalents de LDA, puis on maintient la température à +4°C pendant 30 minutes. On verse le milieu sur une solution saturée de chlorure d'ammonium, extrait, sèche et concentre. Par chromatographie sur silice, en éluant par le chlorure de méthylène, on obtient le produit attendu sous forme d'un mélange de 2 isomères.

m = 2 g

D) N-isopentyl chloro-5 cyclohexyl-3 triméthylsilyloxy-3 tosyl-1 indoline-2 carboxamide.

1 g du produit obtenu précédemment est chauffé à 100°C, sous argon, dans 5 g de HMDS, en présence de 0,1 g d'imidazole pendant 12 heures. On évapore le milieu, reprend par du DCM et chromatographie sur silice. Le DCM élue successivement l'isomère le moins polaire que l'on recristallise dans l'éther iso.

m = 345 mg

Fc = 137-138°C

puis élue l'isomère le plus polaire. On le recristallise dans l'éther iso:

m = 165 mg

Fc = 175-176°C

E) N-isopentyl chloro-5 cyclohexyl-3 hydroxy-3 tosyl-1 indoline-2 carboxamide, isomère cis.

150 mg de l'isomère le moins polaire obtenu précédemment sont traités par 10 mg de soude dans 0°C dans 5 ml de THF et 2 ml d'eau pendant 3 heures. On reprend par de l'eau, extrait au DCM, sèche et concentre. Le brut est recristallisé dans le mélange DCM/éther iso.

m = 120 mg

Fc = 189-191°C.

F) N-isopentyl chloro-5 cyclohexyl-3 hydroxy-3 tosyl-1 indoline-2 carboxamide, isomère trans.

150 mg de l'isomère le plus polaire obtenu à l'étape D sont traités par 10 mg de soude dans 5 ml de THF et 2 ml d'eau, pendant 2 heures à TA. On reprend par de l'eau, extrait au DCM, sèche et concentre. Le produit attendu est recristallisé dans le mélange DCM/éther iso.

m = 65 mg

Fc = 195-196°C

En procédant selon les exemples décrits ci-dessus, on a préparé les composés selon l'invention décrits dans le tableau 2 ci-après :

TABLEAU 2

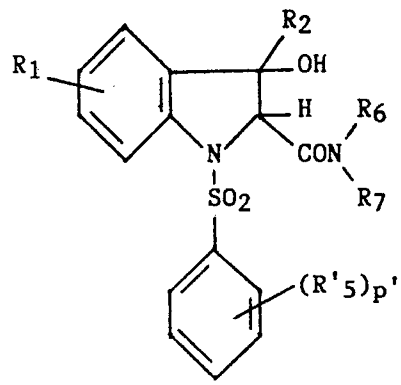

Pour chaque composé de formule (I) ayant les substituants $R_1$, $R_2$, $(R'_5)_{p'}$ et $NR_6R_7$ du tableau ci-dessous, l'isomère cis est indiqué, puis l'isomère trans, sauf indication contraire.

| Ex. | $R_1$ | $R_2$ | $(R'_5)_{p'}$ | $-NR_6R_7$ | Fc °C solvant cristal. |
|---|---|---|---|---|---|
| 115 | Cl-5 | Cl-2 phényle | diméthoxy-3,4 | $N(CH_3)_2$ | 201 DCM-éther iso |
| 116 | | | | | 222 DCM-éther iso |
| 117 | Cl-5 | cyclohexyle | $CH_3$-4 | $NHCH_3$ | 224-225 DCM-éther iso |
| 118 | | | | | 141-148 DCM-éther iso |

| | | | | | | |
|---|---|---|---|---|---|---|
| 119 trans | Cl-5 | Cl-2 phényle | diméthoxy-3,4 | $NHCH_3$ | 148 DCM-éther iso | |
| 120 | Br-5 | F-2 phényle | diméthoxy-3,4 | $N(CH_3)_2$ | 165 DCM-éther iso | |
| 121 | | | | | 214 DCM-éther iso | |
| 122 cis | Cl-5 | Cl-2 phényle | diméthoxy-2,5 | $N(CH_3)_2$ | 140-142 DCM-éther iso | |
| 123 | Cl-5 | Cl-2 phényle | méthoxy-4 | $N(CH_3)_2$ | 240 DCM-hexane | |
| 124 | | | | | 187 DCM-hexane | |
| 125 | Cl-5 | Cl-2 phényle | diméthoxy-3,4 | $N(CH_2CH_3)_2$ | 213 hexane-éther iso | |
| 126 | | | | | 200 hexane-éther iso | |
| 127 | Cl-5 | Cl-2 phényle | diméthoxy-3,4 | $N(CH_3)-CH_2-CH_2-C_6H_5$ | 125-130 hexane-éther iso | |
| 128 | | | | | 140-142 DCM- | |

| | | | | | |
|---|---|---|---|---|---|
| | | | | | éther iso |
| 129 | Cl-5 | Cl-2 phényle | diméthoxy-2,4 | $N(CH_3)_2$ | 213 |
| 130 | | | | | 206 |
| 131 | Cl-5 | méthoxy-2 phényle | diméthoxy-3,4 | $N(CH_3)_2$ | 205 hexane- éther iso |
| 132 | | | | | 206 hexane- éther iso |
| 133 | $CH_3$-5 | Cl-2 phényle | diméthoxy-3,4 | $N(CH_2CH_3)_2$ | 189 éther iso |
| 134 | | | | | 191 éther iso |
| 135 | $CH_3$-5 | Cl-2 phényle | diméthoxy-2,4 | $N(CH_2CH_3)_2$ | 208-209 éther iso |
| 136 | | | | | 214-215 éther iso |
| 137 | Cl-5 | Cl-2 phényle | diméthoxy-2,4 | $N(CH_2CH_3)_2$ | 225 |
| 138 | | | | | 200 |
| 139 | Cl-5 | Cl-2 phényle | cyano-4 | $N(CH_3)_2$ | 242-243 THF, EtOH |
| 140 | | | | | 228-231 DCM- éther iso |

| | | | | | |
|---|---|---|---|---|---|
| 141 cis | Cl-5 | Cl-2 phényle | diméthoxy-3,4 | $N(C_4H_9)_2$ | 203 DCM-éther iso |
| 142 | Cl-5 | cyclohexyle | diméthoxy-3,4 | $N(CH_2CH_3)_2$ | 117-120 |
| 143 | | | | | RMN |
| 144 cis | Cl-5 | F-2 phényle | nitro-4 | $N(CH_3)_2$ | 230 |

Spectre RMN

Exemple 143: isomère trans

0,2-1,8 ppm : m : 17H : $2CH_3$ (éthyle) 11H cyclohexyle

2,8-3,5 ppm : m : 4H : $N-(CH_2)_2-$

3,7 ppm : s : 3H : $OCH_3$

3,75 ppm : s : 3H : $OCH_3$

4,7 ppm : s : 1H : H (indoline)

5,65 ppm : s : 1H : OH (indoline)

6,8-7,6 ppm : m : 6H : aromatiques

EXEMPLES 145 ET 146

chloro-5 (chloro-2 phényl)-3 hydroxy-3 tosyl-1 indoline-2 carboxylate de méthyle : isomère cis destrogyre, isomère cis levogyre.

Les isomères optiques du chloro-5 (chloro-2 phényl)-3 hydroxy-3 tosyl-1 indoline-2 carboxylate de méthyle, décrit à l'exemple 48 du tableau 1 ont été séparés par chromatographie préparative sur colonne chirale.

On effectue tout d'abord une chromatographie analytique en phase supercritique du produit de l'exemple 48.

La colonne utilisée est une colonne Chiralcel OD $^R$ commercialisée par DAICEL. Cette colonne est constituée de gel de silice recouvert de carbamate de cellulose.

L'éluant est un mélange dioxyde de carbone/propanol-2/diéthylamine (75/25/0,3 ; v/v/v) utilisé à un débit de 2 ml/minute . La pression de sortie est de 160 atmosphères, la température est 32 °C et la détection UV est effectuée à 226 nm.

Le chromatogramme présente 2 pics d'égales surfaces et pour des temps de rétention voisins de 4 minutes et 5,4 minutes.

Pour la chromatographie préparative, on utilise également une colonne chiralcel $^R$.

L'échantillon de produit à chromatographier est mis en solution dans le méthanol (30 mg/ml) et 1 ml est injecté dans la colonne. L'éluant est un mélange hexane/propanol-2 (80/20 ; v/v) utilisé avec un débit de 1,5 ml/minute. On effectue une détection UV à 226 nm. Le temps d'analyse est 45 minutes. Douze fractions ont été collectées et analysées en phase supercritique.

Dans les mêmes conditions, on effectue alors 13 injections de 30 mg. Les fractions correspondant au premier pic, recueillies entre 16 et 24 minutes sont regroupées ; les fractions correspondant au deuxième pic, recueillies entre 29 et 42 minutes sont regroupées. Après passage sous un courant d'azote chaque lot est recristallisé dans un mélange DCM/éther iso/hexane.

Le premier pic donne un produit de pureté chromatographique supérieure à 99,9 %, c'est l'isomère cis dextrogyre.

$\alpha_D^{25}$ = +236 (chloroforme)

Fc = 174-177 °C (après recristallisation dans DCM/hexane/éther iso)

m = 130 mg

Le deuxième pic donne un produit de pureté chromatographique supérieur à 99,5 %. C'est l'isomère cis, levogyre

$\alpha_D^{25}$ = -238 (chloroforme)

Fc = 174-177 °C (après recristallisation dans DCM/hexane/éther iso)

m = 83 mg

**Revendications**

**Revendications pour les Etats contractants suivants : DE, GB, FR, IT, NL, SE, CH, LI, BE, AT, LU, DK**

**1.** Un composé de formule :

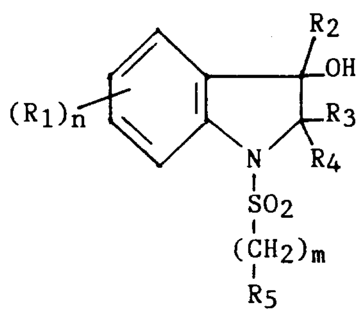

(I)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un alkyle en $C_1$-$C_4$ ; un naphtyl-1 ; un naphtyl-2 ; un phényle non substitué ou substitué par un ou plusieurs substituants choisis parmi un atome d'halogène, un alkyle en $C_1$-$C_4$, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy, un groupe cyano ;
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6 ;

ainsi que ses sels éventuels.

**2.** Composé selon la revendication 1 dans lequel $R_1$ représente un atome de chlore, de brome ou un groupe méthoxy, n = 1 et les autres substituants ont les valeurs définies pour (I) dans la revendication 1.

**3.** Composé selon la revendication 1 dans lequel $R_2$ représente un méthoxyphényle, un chlorophényle ou un cyclohexyle et les autres substituants ont les valeurs définies pour (I) dans la revendication 1.

**4.** Composé selon la revendication 1 dans lequel $R_3$ représente l'hydrogène et les autres substituants ont les valeurs définies pour (I) dans la revendication 1.

**5.** Composé selon la revendication 1 dans lequel $R_4$ représente un alcoxycarbonyle dans lequel le groupe alkyle est en $C_1$-$C_6$ et les autres substituants ont les valeurs définies pour (I) dans la revendication 1.

**6.** Composé selon la revendication 1 dans lequel $R_4$ représente un groupe carboxamide $CONR_6R_7$ dans lequel $R_6$ et $R_7$ sont des alkyles en $C_1$-$C_6$ et les autres substituants ont les valeurs définies pour (I) dans la revendication 1.

**7.** Composé selon la revendication 1 dans lequel $R_5$ représente un phényle substitué en position 3 et 4 ou en position 2 et 4 par un groupe méthoxy, ou bien $R_5$ représente un phényle substitué en position 4 par un groupe méthyle et les autres substituants ont les valeurs définies pour (I) dans la revendication 1.

**8.** Composé selon la revendication 1 dans lequel m est 0, et les autres substituants ont les valeurs définies pour (I) dans la revendication 1.

**9.** Composé selon la revendication 1 sous forme d'isomère cis dans lequel $R_2$ et $R_4$ sont du même côté du cycle indoline.

**10.** Procédé pour la préparation d'un composé selon la revendication 1 caractérisé en ce que :

a) on fait réagir sur un dérivé d'amino-2 phénone de formule:

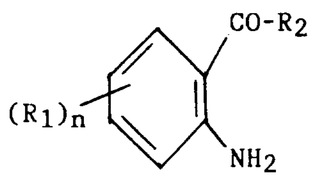

(II)

dans laquelle $R_1$, $R_2$ et n ont les significations indiquées ci-dessus pour I, un dérivé sulfonyle de formule :

$Hal - SO_2 - (CH_2)_m - R_5$ (III)

dans laquelle

- Hal représente un halogène, de préférence le chlore ou le brome,
- et $R_5$ et m ont les significations indiquées pour (I) dans la revendication 1 ;

b) le composé ainsi obtenu de formule :

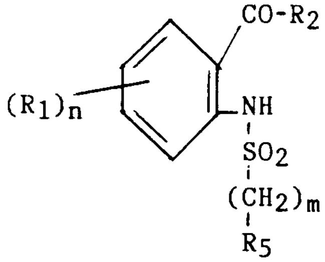

(IV)

est traité par un dérivé halogéné de formule :

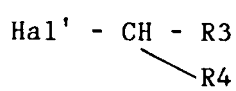

(V)

dans laquelle

Hal' représente un halogène préférentiellement le brome et $R_3$ et $R_4$ ont les significations indiquées pour (I) dans la revendication 1 ;
c) le composé ainsi obtenu de formule :

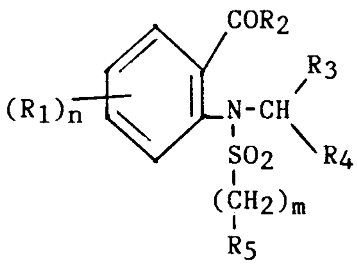
(VI)

est cyclisé en milieu basique pour préparer le composé (I) selon la revendication 1.
d) on sépare éventuellement les isomères cis et trans du composé (I).

**11.** Un composé de formule :

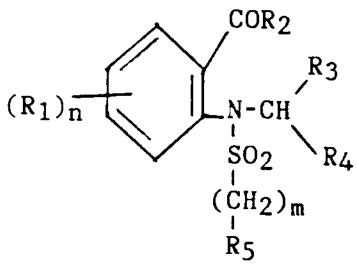
(VI)

dans laquelle
- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un naphtyl-1; un naphtyl-2 ; un phényle substitué par un ou plusieurs substituants choisis parmi un atome d'halogène, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy ou un groupe cyano.
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6.

**12.** Composition pharmaceutique contenant à titre de principe actif un composé selon l'une quelconque des revendications 1 à 9.

**13.** Composition pharmaceutique contenant un composé selon l'une quelconque des revendications 1 à 9 en association avec un autre principe actif.

**Revendications pour l'Etat contractant suivant : GR**

1. Un composé de formule :

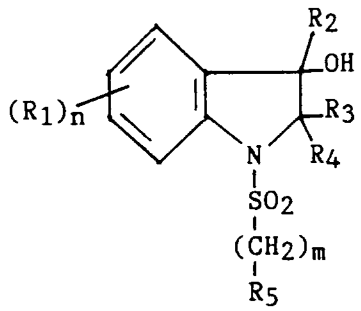

(I)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un alkyle en $C_1$-$C_4$ ; un naphtyl-1 ; un naphtyl-2 ; un phényle non substitué ou substitué par un ou plusieurs substituants choisis parmi un atome d'halogène, un alkyle en $C_1$-$C_4$, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy, un groupe cyano ;
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6 ;

ainsi que ses sels éventuels.

2. Composé selon la revendication 1 dans lequel $R_1$ représente un atome de chlore, de brome ou un groupe méthoxy, n = 1 et les autres substituants ont les valeurs définies pour (I) dans la revendication 1.

3. Composé selon la revendication 1 dans lequel $R_2$ représente un méthoxyphényle, un chlorophényle ou un cyclohexyle et les autres substituants ont les valeurs définies pour (I) dans la revendication 1.

4. Composé selon la revendication 1 dans lequel $R_3$ représente l'hydrogène et les autres substituants ont les valeurs définies pour (I) dans la revendication 1.

5. Composé selon la revendication 1 dans lequel $R_4$ représente un alcoxycarbonyle dans lequel le groupe alkyle est en $C_1$-$C_6$ et les autres substituants ont les valeurs définies pour (I) dans la revendication 1.

6. Composé selon la revendication 1 dans lequel $R_4$ représente un groupe carboxamide $CONR_6R_7$ dans lequel $R_6$ et $R_7$ sont des alkyles en $C_1$-$C_6$ et les autres substituants ont les valeurs définies pour (I) dans la revendication 1.

7. Composé selon la revendication 1 dans lequel $R_5$ représente un phényle substitué en position 3 et 4 ou en position 2 et 4 par un groupe méthoxy, ou bien $R_5$ représente un phényle substitué en position 4 par un groupe méthyle et les autres substituants ont les valeurs définies pour (I) dans la revendication

1.

**8.** Composé selon la revendication 1 dans lequel m est 0, et les autres substituants ont les valeurs définies pour (I) dans la revendication 1.

**9.** Composé selon la revendication 1 sous forme d'isomère cis dans lequel $R_2$ et $R_4$ sont du même côté du cycle indoline.

**10.** Procédé pour la préparation d'un composé selon la revendication 1 caractérisé en ce que :

a) on fait réagir sur un dérivé d'amino-2 phénone de formule:

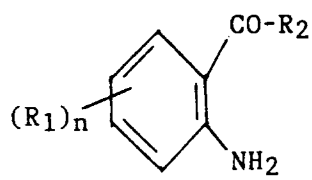

(II)

dans laquelle $R_1$, $R_2$ et n ont les significations indiquées ci-dessus pour I, un dérivé sulfonyle de formule :

$Hal - SO_2 - (CH_2)_m - R_5$ (III)

dans laquelle

- Hal représente un halogène, de préférence le chlore ou le brome,
- et $R_5$ et m ont les significations indiquées pour (I) dans la revendication 1 ;

b) le composé ainsi obtenu de formule :

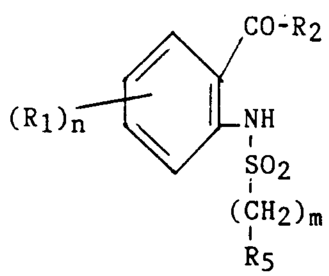

(IV)

est traité par un dérivé halogéné de formule :

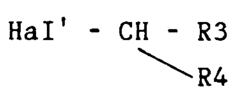

(V)

dans laquelle

Hal' représente un halogène préférentiellement le brome et $R_3$ et $R_4$ ont les significations indiquées pour (I) dans la revendication 1 ;

c) le composé ainsi obtenu de formule :

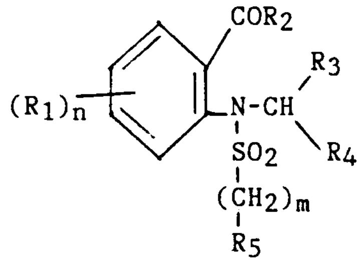

(VI)

est cyclisé en milieu basique pour préparer le composé (I) selon la revendication 1.
d) on sépare éventuellement les isomères <u>cis</u> et <u>trans</u> du composé (I).

**11.** Un composé de formule :

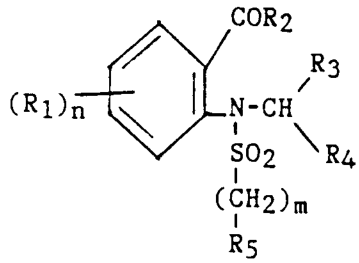

(VI)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un naphtyl-1; un naphtyl-2 ; un phényle substitué par un ou plusieurs substituants choisis parmi un atome d'halogène, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy ou un groupe cyano.
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6.

**12.** Procédé pour la préparation d'une composition pharmaceutique, caractérisé en ce que l'on mélange, en tant que principe actif, un composé selon l'une quelconque des revendications 1 à 9 avec un véhicule pharmaceutiquement acceptable.

**Revendications pour l'Etat contractant suivant : ES**

**1.** Procédé pour la préparation d'un composé de formule :

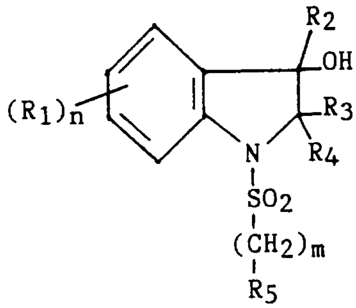

(I)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un alkyle en $C_1$-$C_4$ ; un naphtyl-1 ; un naphtyl-2 ; un phényle non substitué ou substitué par un ou plusieurs substituants choisis parmi un atome d'halogène, un alkyle en $C_1$-$C_4$, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy, un groupe cyano ;
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6 ;

ainsi que ses sels éventuels,
caractérisé en ce que :

a) on fait réagir sur un dérivé d'amino-2 phénone de formule:

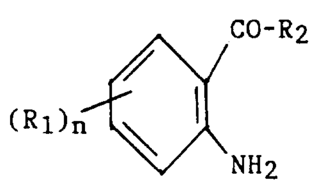

(II)

dans laquelle $R_1$, $R_2$ et n ont les significations indiquées ci-dessus un dérivé sulfonyle de formule :

$Hal - SO_2 - (CH_2)_m - R_5$ (III)

dans laquelle

- Hal représente un halogène, de préférence le chlore ou le brome,
- et $R_5$ et m ont les significations indiquées ci-dessus ;

b) le composé ainsi obtenu de formule :

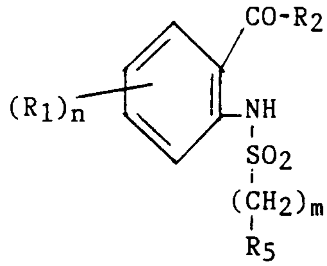

(IV)

est traité par un dérivé halogéné de formule :

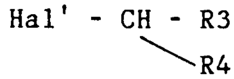

(V)

dans laquelle

Hal' représente un halogène préférentiellement le brome et $R_3$ et $R_4$ ont les significations indiquées ci-dessus ;

c) le composé ainsi obtenu de formule :

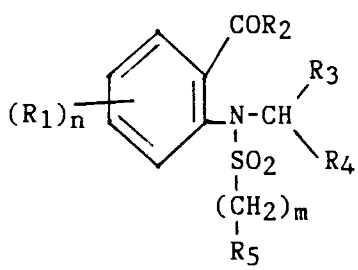

(VI)

est cyclisé en milieu basique pour préparer le composé (I),

d) on sépare éventuellement les isomères cis et trans du composé (I), que l'on transforme éventuellement en l'un de leurs sels.

2. Procédé selon la revendication 1 pour la préparation d'un composé de formule (I) dans laquelle $R_1$ représente un atome de chlore, de brome ou un groupe méthoxy, n = 1 et les autres substituants ont les valeurs définies pour (I) dans la revendication 1.

3. Procédé selon la revendication 1 pour la préparation d'un composé de formule (I) dans laquelle $R_2$ représente un méthoxyphényle, un chlorophényle ou un cyclohexyle et les autres substituants ont les valeurs définies pour (I) dans la revendication 1.

4. Procédé selon la revendication 1 pour la préparation d'un composé de formule (I) dans laquelle $R_3$ représente l'hydrogène et les autres substituants ont les valeurs définies pour (I) dans la revendication 1.

5. Procédé selon la revendication 1 pour la préparation d'un composé de formule (I) dans laquelle $R_4$ représente un alcoxycarbonyle dans lequel le groupe alkyle est en $C_1$-$C_6$ et les autres substituants ont les valeurs définies pour (I) dans la revendication 1.

**6.** Procédé selon la revendication 1 pour la préparation d'un composé de formule (I) dans laquelle $R_4$ représente un groupe carboxamide $CONR_6R_7$ dans lequel $R_6$ et $R_7$ sont des alkyles en $C_1$-$C_6$ et les autres substituants ont les valeurs définies pour (I) dans la revendication 1.

**7.** Procédé selon la revendication 1 pour la préparation d'un composé de formule (I) dans laquelle $R_5$ représente un phényle substitué en position 3 et 4 ou en position 2 et 4 par un groupe méthoxy, ou bien $R_5$ représente un phényle substitué en position 4 par un groupe méthyle et les autres substituants ont les valeurs définies pour (I) dans la revendication 1.

**8.** Procédé selon la revendication 1 pour la préparation d'un composé de formule (I) dans laquelle m est 0 , et les autres substituants ont les valeurs définies pour (I) dans la revendication 1.

**9.** Procédé selon la revendication 1 pour la préparation d'un composé de formule (I) sous forme d'isomère cis dans lequel $R_2$ et $R_4$ sont du même côté du cycle indoline.

**10.** Procédé pour la préparation d'un composé de formule

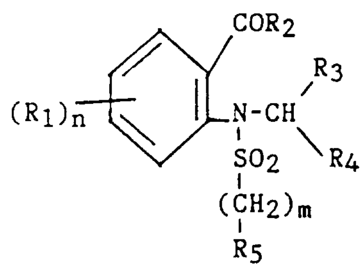

(VI)

dans laquelle

- $R_1$ représente un atome d'halogène, un alkyle en $C_1$-$C_4$, un hydroxyle, un alcoxy en $C_1$-$C_4$, un groupe benzyloxy, un groupe cyano, un groupe trifluorométhyle, un groupe nitro ou un groupe amino ;
- $R_2$ représente un alkyle $C_1$-$C_6$ , un cycloalkyle en $C_3$-$C_7$, un phényle non substitué ou substitué une ou plusieurs fois par un alkyle en $C_1$-$C_4$, un alcoxy en $C_1$-$C_4$, un halogène, un groupe trifluorométhyle ;
- $R_3$ représente un atome d'hydrogène ou un alkyle en $C_1$-$C_4$ ;
- $R_4$ représente un groupe carboxyle, un groupe alcoxycarbonyle dans lequel le groupe alkyle est en $C_1$-$C_6$, un groupe benzyloxycarbonyle ou un groupe carboxamide de formule $CONR_6R_7$ .
- $R_5$ représente un naphtyl-1; un naphtyl-2 ; un phényle substitué par un ou plusieurs substituants choisis parmi un atome d'halogène, un groupe trifluorométhyle, un groupe nitro, un groupe amino libre ou substitué par un ou 2 alkyles en $C_1$-$C_4$, un hydroxyle, un alcoxy en $C_1$-$C_4$, un alcénoxy en $C_1$-$C_4$, un groupe trifluorométhoxy, un groupe benzyloxy ou un groupe cyano,
- $R_6$ et $R_7$ représentent chacun indépendamment l'hydrogène, un alkyle en $C_1$-$C_6$, un phénylalkyle dans lequel l'alkyle est en $C_1$-$C_4$ ou $R_6$ et $R_7$, ensemble constituent un groupement $-(CH_2)_p-$ ;
- n représente 0, 1 ou 2 ;
- m représente 0, 1 ou 2 ;
- p représente 4, 5 ou 6.

**11.** Procédé pour la préparation d'une composition pharmaceutique, caractérisé en ce que l'on mélange, en tant que principe actif, un composé préparé selon le procédé de l'une quelconque des revendications 1 à 9 avec un véhicule pharmaceutiquement acceptable.

**Claims**

**Claims for the following Contracting States : DE, GB, FR, IT, NL, SE, CH, LI, BE, AT, LU, DK**

1. A compound of formula:

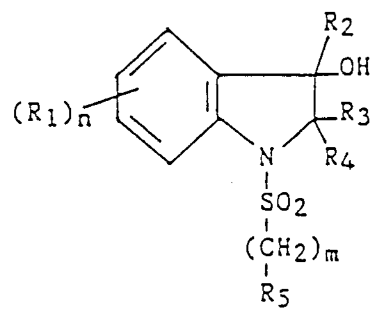

(I)

in which
- $R_1$ is a halogen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl a $C_1$-$C_4$ alkoxy, a halogen, a trifluoromethyl group;
- $R_3$ is a hydrogen atom or a $C_1$-$C_4$ alkyl;
- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a $C_1$-$C_4$ alkyl; a 1-naphthyl; a 2-naphthyl; a phenyl which is unsubstituted or substituted by one or more substituents selected from a halogen atom, a $C_1$-$C_4$ alkyl, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group, a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-(CH_2)_p-$;
- n is 0, 1 or 2;
- m is 0, 1 or 2;
- p is 4, 5 or 6;

and its salts, where appropriate.

2. Compound according to claim 1 in which $R_1$ is a chlorine or bromine atom or a methoxy group, n = 1 and the other substituents are as defined for (I) in claim 1.

3. Compound according to claim 1 in which $R_2$ is a methoxyphenyl, a chlorophenyl or a cyclohexyl and the other substituents are as defined for (I) in claim 1.

4. Compound according to claim 1 in which $R_3$ is a hydrogen and the other substituents are as defined for (I) in claim 1.

5. Compound according to claim 1 in which $R_4$ is an alkoxycarbonyl in which the alkyl group is $C_1$-$C_6$, and the other substituents are as defined for (I) in claim 1.

6. Compound according to claim 1 in which $R_4$ is a carboxamide group $CONR_6R_7$ in which $R_6$ and $R_7$ are $C_1$-$C_6$ alkyls, and the other substituents are as defined for (I) in claim 1.

7. Compound according to claim 1 in which $R_5$ is a phenyl substituted in the 3 and 4 positions or in the 2 and 4 positions by a methoxy group, or else $R_5$ is a phenyl substituted in the 4 position by a methyl group, and the other substituents are as defined for (I) in claim 1.

8. Compound according to claim 1 in which m is 0 and the other substituents are as defined for (I) in claim 1.

**9.** Compound according to claim 1 in the form of the cis isomer in which $R_2$ and $R_4$ are on the same side of the indoline ring.

**10.** Method of preparing a compound according to claim 1, characterized in that

a) a sulfonyl derivative of the formula

$Hal\text{-}SO_2\text{-}(CH_2)_m\text{-}R_5$ (III)

in which

- Hal is a halogen, preferably chlorine or bromine,
- and $R_5$ and m are as defined for (I) in claim 1, is reacted with a 2-aminophenone derivative of the formula

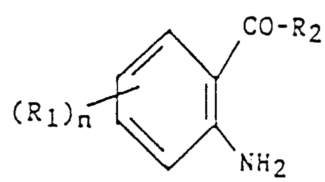

(II)

in which $R_1$, $R_2$ and n are as defined above for (I);

b) the resulting compound of the formula

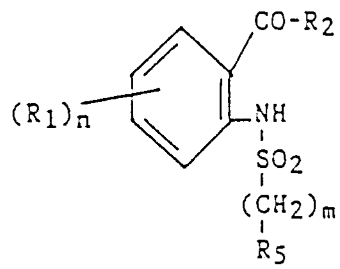

(IV)

is treated with a halogenated derivative of the formula

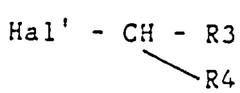

(V)

in which

Hal' is a halogen, preferably bromine, and $R_3$ and $R_4$ are as defined for (I) in claim 1;

c) the resulting compound of the formula

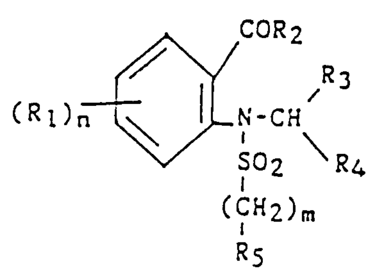

(VI)

is cyclized in a basic medium in order to prepare the compound (I) according to claim 1.
d) the cis and trans isomers of the compound (I) are separated, if appropriate.

**11.** A compound of formula

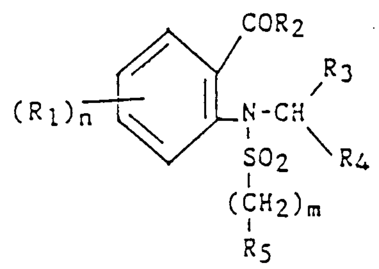

(VI)

in which

- $R_1$ is a halogen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl, a $C_1$-$C_4$ alkoxy, a halogen, a trifluoromethyl group;
- $R_3$ is a hydrogen atom or a $C_1$-$C_4$ alkyl;
- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a 1-naphthyl; a 2-naphthyl; a phenyl which is substituted by one or more substituents selected from a halogen atom, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group or a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-(CH_2)_p-$;
- n is 0, 1 or 2;
- m is 0, 1 or 2;
- p is 4, 5 or 6.

**12.** Pharmaceutical composition in which a compound according to any one of claims 1 to 9 is present as the active principle.

**13.** Pharmaceutical composition in which a compound according to any one of claims 1 to 9 is present in association with another active principle.

**Claims for the following Contracting State : GR**

**1.** A compound of formula:

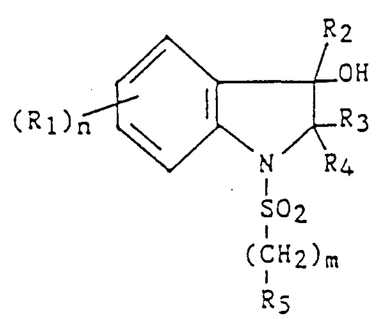

(I)

in which

- $R_1$ is a haloglen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl, a $C_1$-$C_4$ alkoxy, a halogen, a trifluoromethyl group;
- $R_3$ is a hydrogen atom or a $C_1$-$C_4$ alkyl;
- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a $C_1$-$C_4$ alkyl; a 1-naphthyl; a 2-naphthyl; a phenyl which is unsubstituted or substituted by one or more substituents selected from a halogen atom, a $C_1$-$C_4$ alkyl, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group, a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-CH_2)_p-$;
- n is 0, 1 or 2;
- m is 0, 1 or 2;
- p is 4, 5 or 6;

and its salts, where appropriate.

**2.** Compound according to claim 1 in which $R_1$ is a chlorine or bromine atom or a methoxy group, n = 1 and the other substituents are as defined for (I) in claim 1.

**3.** Compound according to claim 1 in which $R_2$ is a methoxyphenyl, a chlorophenyl or a cyclohexyl and the other substituents are as defined for (I) in claim 1.

**4.** Compound according to claim 1 in which $R_3$ is a hydrogen and the other substituents are as defined for (I) in claim 1.

**5.** Compound according to claim 1 in which $R_4$ is an alkoxycarbonyl in which the alkyl group is $C_1$-$C_6$, and the other substituents are as defined for (I) in claim 1.

**6.** Compound according to claim 1 in which $R_4$ is a carboxamide group $CONR_6R_7$ in which $R_6$ and $R_7$ are $C_1$-$C_6$ alkyls, and the other substituents are as defined for (I) in claim 1.

**7.** Compound according to claim 1 in which $R_5$ is a phenyl substituted in the 3 and 4 positions or in the 2 and 4 positions by a methoxy group, or else $R_5$ is a phenyl substituted in the 4 position by a methyl group, and the other substituents are as defined for (I) in claim 1.

**8.** Compound according to claim 1 in which m is 0 and the other substituents are as defined for (I) in claim 1.

**9.** Compound according to claim 1 in the form of the cis isomer in which $R_2$ and $R_4$ are on the same side of the indoline ring.

**10.** Method of preparing a compound according to claim 1, characterized in that

a) a sulfonyl derivative of the formula

$Hal-SO_2-(CH_2)_m-R_5$ (III)

in which

- Hal is a halogen, preferably chlorine or bromine,
- and $R_5$ and m are as defined for (I) in claim 1, is reacted with a 2-aminophenone derivative of the formula

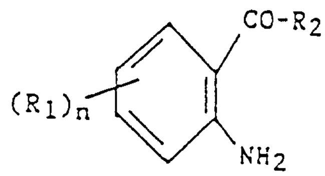

(II)

in which $R_1$, $R_2$ and n are as defined above for (I);

b) the resulting compound of the formula

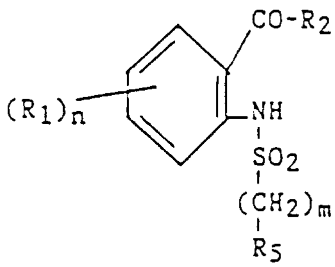

(IV)

is treated with a halogenated derivative of the formula

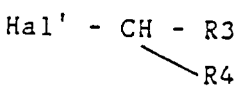

(V)

in which

Hal' is a halogen, preferably bromine, and $R_3$ and $R_4$ are as defined for (I) in claim 1;

c) the resulting compound of the formula

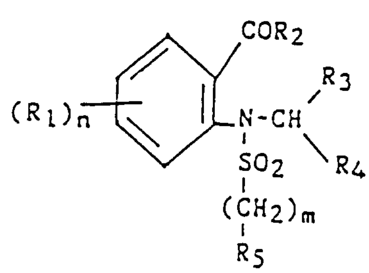

(VI)

is cyclized in a basic medium in order to prepare the compound (I) according to claim 1.

d) the cis and trans isomers of the compound (I) are separated, if appropriate.

**11.** A compound of formula

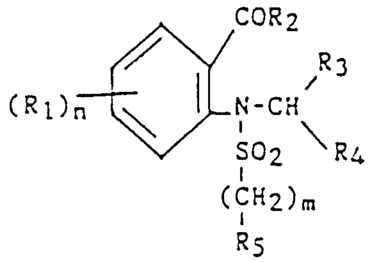

(VI)

in which

- $R_1$ is a halogen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl, a $C_1$-$C_4$ alkoxy, a halogen, a trifluoromethyl group;
- $R_3$ is a hydrogen atom or a $C_1$-$C_4$ alkyl;
- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a 1-naphthyl; a 2-naphthyl; a phenyl which is substituted by one or more substituents selected from a halogen atom, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group or a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-CH_2)_p-$;
- n is 0, 1 or 2;
- m is 0, 1 or 2;
- p is 4, 5 or 6.

**12.** Method for the preparation of a pharmaceutical composition, characterized in that a compound according to any one of claims 1 to 9 is mixed, as the active principle, with a pharmaceutically acceptable vehicle.

**Claims for the following Contracting State : ES**

**1.** Method of preparing a compound of formula:

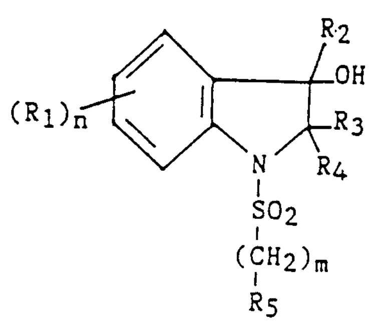

(I)

in which

- $R_1$ is a halogen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl, a $C_1$-$C_4$ alkoxy, a halogen, a trifluoromethyl group;
- $R_3$ is a hydrogen atom or a $C_1$-$C_4$ alkyl;

- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a $C_1$-$C_4$ alkyl; a 1-naphthyl; a 2-naphthyl; a phenyl which is unsubstituted or substituted by one or more substituents selected from a halogen atom, a $C_1$-$C_4$ alkyl, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group, a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-(CH_2)_p-$;
- n is 0, 1 or 2;
- m is 0, 1 or 2;
- p is 4, 5 or 6;

and its salts, where appropriate,
characterized in that

a) a sulfonyl derivative of the formula

$Hal$-$SO_2$-$(CH_2)_m$-$R_5$ (III)

in which

- Hal is a halogen, preferably chlorine or bromine,
- and $R_5$ and m are as defined above, is reacted with a 2-aminophenone derivative of the formula

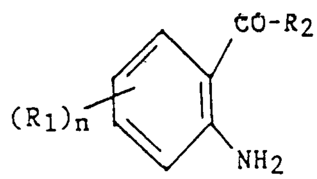

(II)

in which $R_1$, $R_2$ and n are as defined above;

b) the resulting compound of the formula

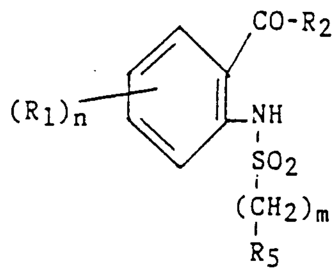

(IV)

is treated with a halogenated derivative of the formula

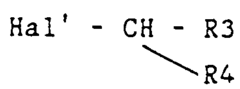

(V)

in which
Hal' is a halogen, preferably bromine, and $R_3$ and $R_4$ are as defined above;

c) the resulting compound of the formula

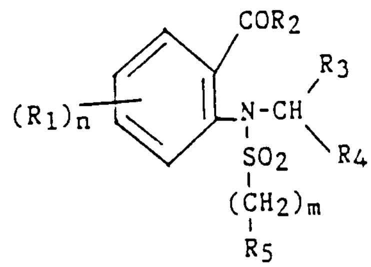

(VI)

is cyclized in a basic medium in order to prepare the compound (I),

d) the cis and trans isomers of the compound (I) are separated, if appropriate, and are optionally converted to one of the salts thereof.

**2.** Method according to claim 1 for the preparation of a compound of formula (I) in which $R_1$ is a chlorine or bromine atom or a methoxy group, n = 1 and the other substituents are as defined for (I) in claim 1.

**3.** Method according to claim 1 for the preparation of a compound of formula (I) in which $R_2$ is a methoxyphenyl, a chlorophenyl or a cyclohexyl and the other substituents are as defined for (I) in claim 1.

**4.** Method according to claim 1 for the preparation of a compound of formula (I) in which $R_3$ is a hydrogen and the other substituents are as defined for (I) in claim 1.

**5.** Method according to claim 1 for the preparation of a compound of formula (I) in which $R_4$ is an alkoxycarbonyl in which the alkyl group is $C_1$-$C_6$, and the other substituents are as defined for (I) in claim 1.

**6.** Method according to claim 1 for the preparation of a compound of formula (I) in which $R_4$ is a carboxamide group $CONR_6R_7$ in which $R_6$ and $R_7$ are $C_1$-$C_6$ alkyls, and the other substituents are as defined for (I) in claim 1.

**7.** Method according to claim 1 for the preparation of a compound of formula (I) in which $R_5$ is a phenyl substituted in the 3 and 4 positions or in the 2 and 4 positions by a methoxy group, or else $R_5$ is a phenyl substituted in the 4 position by a methyl group, and the other substituents are as defined for (I) in claim 1.

**8.** Method according to claim 1 for the preparation of a compound of formula (I) in which m is 0 and the other substituents are as defined for (I) in claim 1.

**9.** Method according to claim 1 for the preparation of a compound of formula (I) in the form of the cis isomer in which $R_2$ and $R_4$ are on the same side of the indoline ring.

**10.** Method of preparing a compound of formula

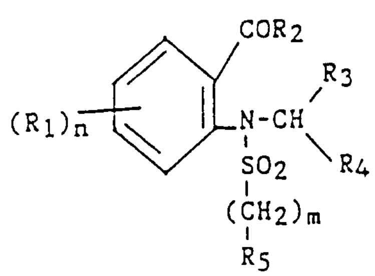

(VI)

in which

- $R_1$ is a halogen atom, a $C_1$-$C_4$ alkyl, a hydroxyl, a $C_1$-$C_4$ alkoxy, a benzyloxy group, a cyano group, a trifluoromethyl group, a nitro group or an amino group;
- $R_2$ is a $C_1$-$C_6$ alkyl, a $C_3$-$C_7$ cycloalkyl, a phenyl which is unsubstituted or monosubstituted or polysubstituted by a $C_1$-$C_4$ alkyl, a $C_1$-$C_4$ alkoxy, a halogen, a trifluoromethyl group;
- $R_3$ is a hydrogen atom or a $C_1$-$C_4$ alkyl;
- $R_4$ is a carboxyl group, an alkoxycarbonyl group in which the alkyl group is $C_1$-$C_6$, a benzyloxycarbonyl group or a carboxamide group of the formula $CONR_6R_7$;
- $R_5$ is a 1-naphthyl; a 2-naphthyl; a phenyl which is substituted by one or more substituents selected from a halogen atom, a trifluoromethyl group, a nitro group, an amino group which is free or substituted by one or 2 $C_1$-$C_4$ alkyls, a hydroxyl, a $C_1$-$C_4$ alkoxy, a $C_1$-$C_4$ alkenoxy, a trifluoromethoxy group, a benzyloxy group or a cyano group;
- $R_6$ and $R_7$ are each independently hydrogen, a $C_1$-$C_6$ alkyl, a phenylalkyl in which the alkyl is $C_1$-$C_4$, or $R_6$ and $R_7$ together form a group $-(CH_2)_p-$;
- n is 0, 1 or 2;
- m is 0, 1 or 2;
- p is 4, 5 or 6.

**11.** Method for the preparation of a pharmaceutical composition, characterized in that a compound according to any one of claims 1 to 9 is mixed, as the active principle, with a pharmaceutically acceptable vehicle.

**Patentansprüche**
**Patentansprüche für folgende Vertragsstaaten : DE, GB, FR, IT, NL, SE, CH, LI, BE, AT, LU, DK**

**1.** Verbindungen der Formel

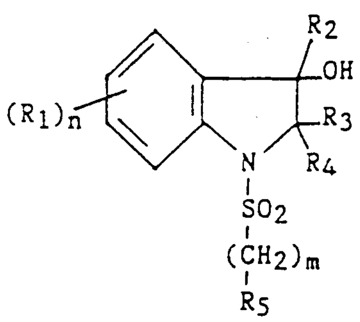

(I),

in der bedeuten:

- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_1$-$C_6$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ $C_{1-4}$-Alkyl, 1-Naphthyl, 2-Naphthyl oder Phenyl, das ggfs. mit einem oder mehreren Substituenten substituiert ist, die ausgewählt sind unter Halogenatomen, $C_{1-4}$-Alkyl, Trifluormethyl, Nitro, freiem Amino, mit einer oder zwei $C_{1-4}$-Alkylgruppen substituiertem Amino, Hydroxy, $C_{1-4}$-Alkoxy, $C_{2-4}$-Alkenyloxy, Trifluormethoxy, Benzyloxy und Cyano,
- n 0, 1 oder 2, und
- m 0, 1 oder 2,

sowie ihre Salze.

**2.** Verbindungen nach Anspruch 1, bei denen $R_1$ ein Chlor- oder Bromatom oder Methoxy, n den Wert 1 und die übrigen Substituenten dasselbe wie für die Verbindungen (I) in Anspruch 1 definiert bedeuten.

**3.** Verbindungen nach Anspruch 1, bei denen $R_2$ Methoxyphenyl, Chlorphenyl oder Cyclohexyl und die übrigen Substituenten dasselbe wie für die Verbindungen (I) in Anspruch 1 definiert bedeuten.

**4.** Verbindungen nach Anspruch 1, bei denen $R_3$ Wasserstoff und die übrigen Substituenten dasselbe wie für die Verbindungen (I) in Anspruch 1 definiert bedeuten.

**5.** Verbindungen nach Anspruch 1, bei denen $R_4$ Alkoxycarbonyl mit $C_{1-6}$-Alkyl und die übrigen Substituenten dasselbe wie für die Verbindungen (I) in Anspruch 1 definiert bedeuten.

**6.** Verbindungen nach Anspruch 1, bei denen $R_4$ eine Carboxamidgruppe $CONR_6R_7$, in der $R_6$ und $R_7$ $C_{1-6}$-Alkylgruppen darstellen, und die übrigen Substituenten dasselbe wie für die Verbindungen (I) in Anspruch 1 definiert bedeuten.

**7.** Verbindungen nach Anspruch 1, bei denen $R_5$ Phenyl, das in den Stellungen 3 und 4 oder den Stellungen 2 und 4 mit Methoxy substituiert ist, oder Phenyl, das in 4-Stellung mit Methyl substituiert ist, und die übrigen Substituenten dasselbe wie für die Verbindungen (I) in Anspruch 1 definiert bedeuten.

**8.** Verbindungen nach Anspruch 1, bei denen m gleich Null ist und die übrigen Substituenten dasselbe wie für die Verbindungen (I) in Anspruch 1 definiert bedeuten.

**9.** Verbindungen nach Anspruch 1 in Form der cis-Isomeren, bei dem sich die Substituenten $R_2$ und $R_4$ auf der gleichen Seite des Indolinrings befinden.

**10.** Verfahren zur Herstellung der Verbindungen nach Anspruch 1,
gekennzeichnet durch

a) Umsetzung eines 2-Aminophenons der Formel

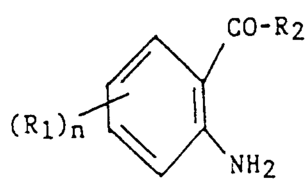

(II),

in der $R_1$, $R_2$ und n dasselbe wie oben für die Verbindungen (I) definiert bedeuten,
mit einem Sulfonylderivat der Formel

$Hal - SO_2 - (CH_2)_m - R_5$ (III),

in der bedeuten:

- Hal ein Halogen, vorzugsweise Chlor oder Brom, und
- $R_5$ und m dasselbe wie für die Verbindungen (I) in Anspruch 1 definiert bedeuten,

b) Behandlung der so erhaltenen Verbindung der Formel

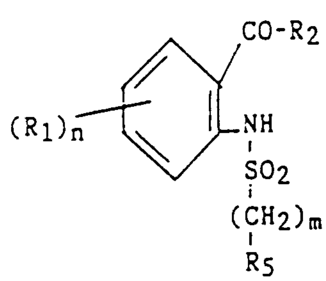

(IV)

mit einem Halogenderivat der Formel

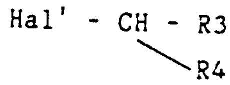

(V),

in der

Hal' ein Halogen, vorzugsweise Brom, und

$R_3$ und $R_4$ dasselbe wie für die Verbindungen (I) in Anspruch 1 definiert bedeuten,

und

c) Cyclisierung der so erhaltenen Verbindung der Formel

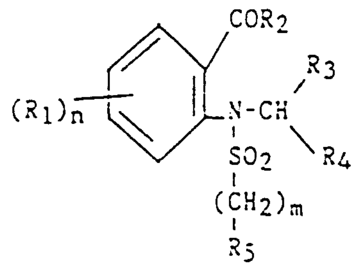

(VI)

in einem basischen Medium unter Erhalt der Verbindung (I) nach Anspruch 1 sowie gegebenenfalls

d) Trennung des cis- und des trans-Isomers der Verbindung (I).

**11.** Verbindungen der Formel

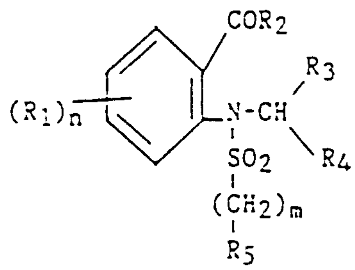

(VI),

in der bedeuten:

- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_{1-6}$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ 1-Naphthyl, 2-Naphthyl oder Phenyl, das mit einem oder mehreren Substituenten substituiert ist, die ausgewählt sind unter Halogenatomen, Trifluormethyl, Nitro, freiem Amino, mit einer oder zwei $C_{1-4}$-Alkylgruppen substituiertem Amino, Hydroxy, $C_{1-4}$-Alkyoxy, $C_{2-4}$-Alkenyloxy, Trifluormethoxy, Benzyloxy und Cyano,
- n 0, 1 oder 2, und
- m 0, 1 oder 2.

**12.** Pharmazeutische Zusammensetzungen, die als Wirkstoff eine Verbindung nach einem der Ansprüche 1 bis 9 enthalten.

**13.** Pharmazeutische Zusammensetzungen, die eine Verbindung nach einem der Ansprüche 1 bis 9 zusammen mit einem weiteren Wirkstoff enthalten.

**Patentansprüche für folgenden Vertragsstaat : GR**

**1.** Verbindungen der Formel

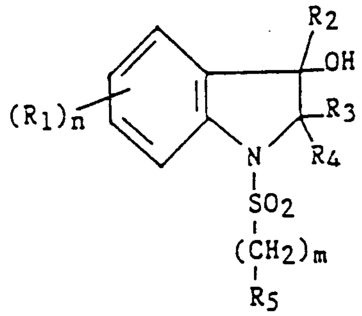

(I),

in der bedeuten:

- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_{1-6}$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ $C_{1-4}$-Alkyl, 1-Naphthyl, 2-Naphthyl oder Phenyl, das ggfs. mit einem oder mehreren Substituenten substituiert ist, die ausgewählt sind unter Halogenatomen, $C_{1-4}$-Alkyl, Trifluormethyl, Nitro, freiem Amino, mit einer oder zwei $C_{1-4}$-Alkylgruppen substituiertem Amino, Hydroxy, $C_{1-4}$-Alkoxy, $C_{2-4}$-Alkenyloxy, Trifluormethoxy, Benzyloxy und Cyano,
- n 0, 1 oder 2, und
- m 0, 1 oder 2,

sowie ihre Salze.

**2.** Verbindungen nach Anspruch 1, bei denen $R_1$ ein Chlor- oder Bromatom oder Methoxy, n den Wert 1 und die übrigen Substituenten dasselbe wie für die Verbindungen (I) in Anspruch 1 definiert bedeuten.

**3.** Verbindungen nach Anspruch 1, bei denen $R_2$ Methoxyphenyl, Chlorphenyl oder Cyclohexyl und die übrigen Substituenten dasselbe wie für die Verbindungen (I) in Anspruch 1 definiert bedeuten.

**4.** Verbindungen nach Anspruch 1, bei denen $R_3$ Wasserstoff und die übrigen Substituenten dasselbe wie für die Verbindungen (I) in Anspruch 1 definiert bedeuten.

**5.** Verbindungen nach Anspruch 1, bei denen $R_4$ Alkoxycarbonyl mit $C_{1-6}$-Alkyl und die übrigen Substituenten dasselbe wie für die Verbindungen (I) in Anspruch 1 definiert bedeuten.

**6.** Verbindungen nach Anspruch 1, bei denen $R_4$ eine Carboxamidgruppe $CONR_6R_7$, in der $R_6$ und $R_7$ $C_{1-6}$-Alkylgruppen darstellen, und die übrigen Substituenten dasselbe wie für die Verbindungen (I) in Anspruch 1 definiert bedeuten.

7. Verbindungen nach Anspruch 1, bei denen $R_5$ Phenyl, das in den Stellungen 3 und 4 oder den Stellungen 2 und 4 mit Methoxy substituiert ist, oder Phenyl, das in 4-Stellung mit Methyl substituiert ist, und die übrigen Substituenten dasselbe wie für die Verbindungen (I) in Anspruch 1 definiert bedeuten.

8. Verbindungen nach Anspruch 1, bei denen m gleich Null ist und die übrigen Substituenten dasselbe wie für die Verbindungen (I) in Anspruch 1 definiert bedeuten.

9. Verbindungen nach Anspruch 1 in Form der cis-Isomeren, bei dem sich die Substituenten $R_2$ und $R_4$ auf der gleichen Seite des Indolinrings befinden.

10. Verfahren zur Herstellung der Verbindungen nach Anspruch 1, gekennzeichnet durch

a) Umsetzung eines 2-Aminophenons der Formel

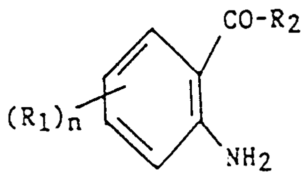

(II),

in der $R_1$, $R_2$ und n dasselbe wie oben für die Verbindungen (I) definiert bedeuten, mit einem Sulfonylderivat der Formel

$Hal - SO_2 - (CH_2)_m - R_5$ (III),

in der bedeuten:

- Hal ein Halogen, vorzugsweise Chlor oder Brom, und
- $R_5$ und m dasselbe wie für die Verbindungen (I) in Anspruch 1 definiert bedeuten,

b) Behandlung der so erhaltenen Verbindung der Formel

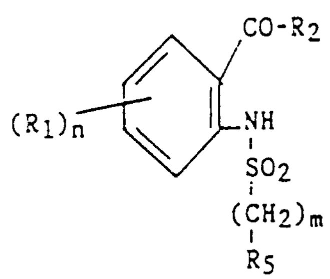

(IV)

mit einem Halogenderivat der Formel

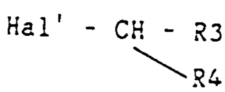

(V),

in der

Hal' ein Halogen, vorzugsweise Brom, und

$R_3$ und $R_4$ dasselbe wie für die Verbindungen (I) in Anspruch 1 definiert bedeuten,

und

c) Cyclisierung der so erhaltenen Verbindung der Formel

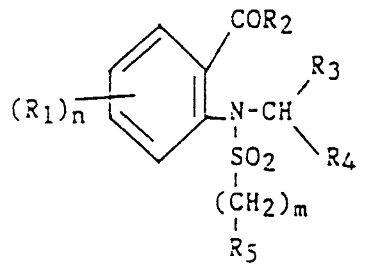

(VI)

in einem basischen Medium unter Erhalt der Verbindung (I) nach Anspruch 1 sowie gegebenenfalls

d) Trennung des cis- und des trans-Isomers der Verbindung (I).

**11.** Verbindungen der Formel

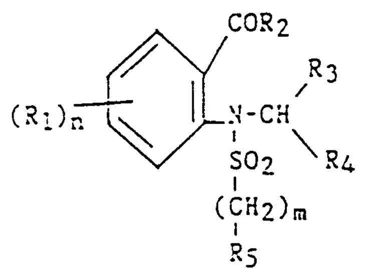

(VI),

in der bedeuten:

- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_{1-6}$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ 1-Naphthyl, 2-Naphthyl oder Phenyl, das mit einem oder mehreren Substituenten substituiert ist, die ausgewählt sind unter Halogenatomen, Trifluormethyl, Nitro, freiem Amino, mit einer oder zwei $C_{1-4}$-Alkylgruppen substituiertem Amino, Hydroxy, $C_{1-4}$-Alkoxy, $C_{2-4}$-Alkenyloxy, Trifluormethoxy, Benzyloxy und Cyano,
- n 0, 1 oder 2, und
- m 0, 1 oder 2.

**12.** Verfahren zur Herstellung von pharmazeutischen Zusammensetzungen, gekennzeichnet durch Mischen einer Verbindung nach einem der Ansprüche 1 bis 9 als Wirkstoff mit einem pharmazeutisch akzeptablen Träger.

**Patentansprüche für folgenden Vertragsstaat : ES**

1. Verfahren zur Herstellung von Verbindungen der Formel

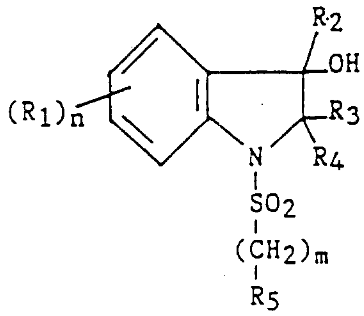

(I),

in der bedeuten:
- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_{1-6}$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ $C_{1-4}$-Alkyl, 1-Naphthyl, 2-Naphthyl oder Phenyl, das ggfs. mit einem oder mehreren Substituenten substituiert ist, die ausgewählt sind unter Halogenatomen, $C_{1-4}$-Alkyl, Trifluormethyl, Nitro, freiem Amino, mit einer oder zwei $C_{1-4}$-Alkylgruppen substituiertem Amino, Hydroxy, $C_{1-4}$-Alkoxy, $C_{2-4}$-Alkenyloxy, Trifluormethoxy, Benzyloxy und Cyano,
- n 0, 1 oder 2, und
- m 0, 1 oder 2,

sowie ihrer ggfs. vorliegenden Salze,
gekennzeichnet durch

a) Umsetzung eines 2-Aminophenons der Formel

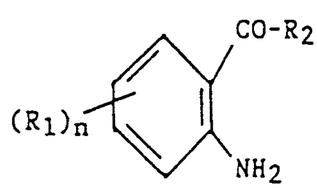

(II),

in der $R_1$, $R_2$ und n dasselbe wie oben für die Verbindungen (I) definiert bedeuten, mit einem Sulfonylderivat der Formel

Hal - $SO_2$ - $(CH_2)_m$ - $R_5$ (III),

in der bedeuten:
- Hal ein Halogen, vorzugsweise Chlor oder Brom, und
- $R_5$ und m dasselbe wie für die Verbindungen (I) in Anspruch 1 definiert bedeuten,

b) Behandlung der so erhaltenen Verbindung der Formel

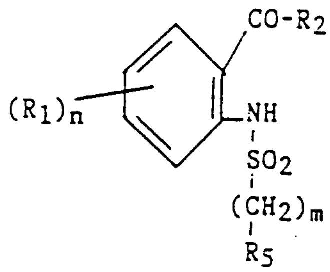

(IV)

mit einem Halogenderivat der Formel

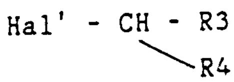

(V),

in der

Hal' ein Halogen, vorzugsweise Brom, und

$R_3$ und $R_4$ dasselbe wie für die Verbindungen (I) in Anspruch 1 definiert bedeuten,

und

c) Cyclisierung der so erhaltenen Verbindung der Formel

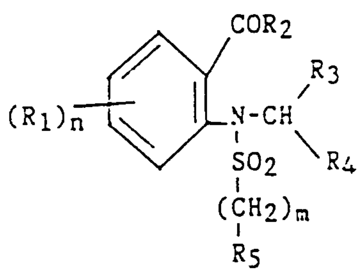

(VI)

in einem basischen Medium unter Erhalt der Verbindung (I) nach Anspruch 1,

sowie gegebenenfalls

d) Trennung des cis- und des trans-Isomers der Verbindung (I)

sowie gegebenenfalls Umwandlung in eines ihrer Salze.

**2.** Verfahren nach Anspruch 1 zur Herstellung von Verbindungen der Formel (I), in der $R_1$ ein Chlor- oder Bromatom oder Methoxy, n den Wert 1 und die übrigen Substituenten dasselbe wie für die Verbindungen (I) in Anspruch 1 definiert bedeuten.

**3.** Verfahren nach Anspruch 1 zur Herstellung von Verbindungen der Formel (I), in der $R_2$ Methoxyphenyl, Chlorphenyl oder Cyclohexyl und die übrigen Substituenten dasselbe wie für die Verbindungen (I) in Anspruch 1 definiert bedeuten.

**4.** Verfahren nach Anspruch 1 zur Herstellung von Verbindungen der Formel (I), in der $R_3$ Wasserstoff und die übrigen Substituenten dasselbe wie für die Verbindungen (I) in Anspruch 1 definiert bedeuten.

**5.** Verfahren nach Anspruch 1 zur Herstellung von Verbindungen der Formel (I), in der $R_4$ Alkoxycarbonyl mit $C_{1-6}$-Alkyl und die übrigen Substituenten dasselbe wie für die Verbindungen (I) in Anspruch 1 definiert bedeuten.

**6.** Verfahren nach Anspruch 1 zur Herstellung von Verbindungen der Formel (I), in der $R_4$ eine Carboxamidgruppe $CONR_6R_7$, in der $R_6$ und $R_7$ $C_{1-6}$-Alkylgruppen darstellen, und die übrigen Substituenten dasselbe wie für die Verbindungen (I) in Anspruch 1 definiert bedeuten.

**7.** Verfahren nach Anspruch 1 zur Herstellung von Verbindungen der Formel (I), in der $R_5$ Phenyl, das in den Stellungen 3 und 4 oder den Stellungen 2 und 4 mit Methoxy substituiert ist, oder Phenyl, das in 4-Stellung mit Methyl substituiert ist, und die übrigen Substituenten dasselbe wie für die Verbindungen (I) in Anspruch 1 definiert bedeuten.

**8.** Verfahren nach Anspruch 1 zur Herstellung von Verbindungen der Formel (I), in der m gleich Null ist, wobei die übrigen Substituenten dasselbe wie für die Verbindungen (I) in Anspruch 1 definiert bedeuten.

**9.** Verfahren nach Anspruch 1 zur Herstellung von Verbindungen der Formel (I) in Form der cis-Isomeren, bei dem sich die Substituenten $R_2$ und $R_4$ auf der gleichen Seite des Indolinrings befinden.

**10.** Verfahren zur Herstellung von Verbindungen der Formel

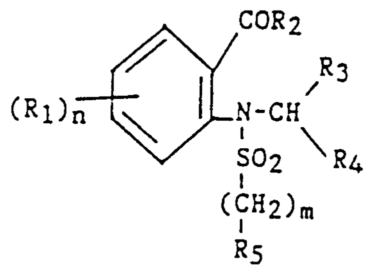

(VI),

in der bedeuten:

- $R_1$ ein Halogenatom, $C_{1-4}$-Alkyl, Hydroxy, $C_{1-4}$-Alkoxy, Benzyloxy, Cyano, Trifluormethyl, Nitro oder Amino,
- $R_2$ $C_{1-6}$-Alkyl, $C_{3-7}$-Cycloalkyl oder Phenyl, das ggfs. mit einem oder mehreren Substituenten substituiert ist, die ausgewählt sind unter $C_{1-4}$-Alkyl, $C_{1-4}$-Alkoxy, Halogenatomen und Trifluormethyl,
- $R_3$ Wasserstoff oder $C_{1-4}$-Alkyl,
- $R_4$ Carboxy, Alkoxycarbonyl mit $C_{1-6}$-Alkyl, Benzyloxycarbonyl oder Carboxamido der Formel $CONR_6R_7$, worin $R_6$ und $R_7$ entweder jeweils unabhängig Wasserstoff, $C_{1-6}$-Alkyl oder Phenylalkyl mit $C_{1-4}$-Alkyl oder $R_6$ und $R_7$ zusammen eine Gruppe $-(CH_2)_p$-bedeuten, worin p 4, 5 oder 6 darstellt,
- $R_5$ 1-Naphthyl, 2-Naphthyl oder Phenyl, das mit einem oder mehreren Substituenten substituiert ist, die ausgewählt sind unter Halogenatomen, Trifluormethyl, Nitro, freiem Amino, mit einer oder zwei $C_{1-4}$-Alkylgruppen substituiertem Amino, Hydroxy, $C_{1-4}$-Alkoxy, $C_{2-4}$-Alkenyloxy, Trifluormethoxy, Benzyloxy und Cyano,
- n 0, 1 oder 2, und
- m 0, 1 oder 2.

**11.** Verfahren zur Herstellung von pharmazeutischen Zusammensetzungen, gekennzeichnet durch Mischen einer Verbindung nach einem der Ansprüche 1 bis 9 als Wirkstoff mit einem pharmazeutisch akzeptablen Träger.